(19) Europäisches Patentamt
European Patent Office
Office européen des brevets

(11) Veröffentlichungsnummer: **0 300 960 B1**

(12) # EUROPÄISCHE PATENTSCHRIFT

(45) Veröffentlichungstag der Patentschrift: **21.12.94**

(51) Int. Cl.$^{5}$: **A61K 31/70**, C07H 15/10

(21) Anmeldenummer: **88810434.6**

(22) Anmeldetag: **24.06.88**

(54) **Neue pharmazeutische Präparate sowie neue Lactosylverbindungen und ihre Herstellung.**

(30) Priorität: **26.06.87 CH 2407/87**

(43) Veröffentlichungstag der Anmeldung:
**25.01.89 Patentblatt 89/04**

(45) Bekanntmachung des Hinweises auf die Patenterteilung:
**21.12.94 Patentblatt 94/51**

(84) Benannte Vertragsstaaten:
**AT BE CH DE ES FR GB GR IT LI LU NL SE**

(56) Entgegenhaltungen:
**EP-A- 0 133 170**
**EP-A- 0 146 810**
**WO-A-81/03175**

**CHEMICAL ABSTRACTS, vol. 91, no. 25, 17th December 1979, page 55, abstract no. 204467w, Columbus, Ohio, US; C.R. ALVING et al.: "Sporozoite-induced malaria: therapeutic effects of glycolipids in liposomes", & SCIENCE, (Washington, DC), 1979, 205(4411), 1142-4**

(73) Patentinhaber: **Solco Basel AG**
**Gellertstrasse 18**
**CH-4052 Basel (CH)**

(72) Erfinder: **Schmidt, Richard R.**
**Grossherzog-Friedrich-Strasse 11**
**D-7750 Konstanz 16 (DE)**
Erfinder: **Baer, Thomas,**
**Alemannenstrasse 13**
**D-7750 Konstanz (DE)**
Erfinder: **Zimmermann, Peter**
**Ruhesteinweg 14**
**D-7730 Villingen (DE)**
Erfinder: **Wendel, Albrecht**
**Im Buckenloh 19**
**D-7400 Tübingen (DE)**

(74) Vertreter: **Frossard, Michel, Dr. et al**
**A. Braun, Braun, Héritier, Eschmann AG,**
**Patentanwälte**
**Holbeinstrasse 36-38**
**CH-4051 Basel (CH)**

EP 0 300 960 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

CHEMICAL ABSTRACTS, vol. 101, no. 12, 17th September 1984, page 407, abstract no. 97595n, Columbus, Ohio, US; H.H. SPANJER et al.: "Lactosylceramide-induced stimulation of liposome uptake by Kupffer cells in vivo", & BIOCHIM. BIOPHYS. ACTA, 1984, 774(1), 49-55

ANGEW. CHEM. INT. ED. ENGL., Band 26, Nr. 8, 1987, Seiten 793-794, VCH Verlagsgesellschaft mbH, Weinheim, DE; R.R. SCHMIDT et al.: "Lactosylceramides with unsaturated fatty acids-synthesis and use in the generation of bilayer membranes"

ANGEW. CHEM., Band 98, Nr. 8, 1986, Seiten 722-723, VCH Verlagsgesellschaft mbH, Weinheim, DE; R.R. SCHMIDT et al.: "Synthese von Glycosphinogolipiden und Psychosinen"

TETRAHEDRON LETTERS, Band 27, Nr. 4, 1986, Seiten 481-484, Pergamon Press Ltd, GB; R.R. SCHMIDT et al.: "Synthesis of D-erythro-sphingosines"

## Beschreibung

Als Cerebroside bezeichnet man eine Gruppe von Glykolipiden, welche aus Ceramiden und Galactose oder Glucose aufgebaut sind und in der Hirnsubstanz und den Nervenzellen vorkommen [Sandhoff, Angew. Chem. 89 (1977), 283-295].

Besonders eingehend untersucht worden sind Glucosylceramide der Formel:

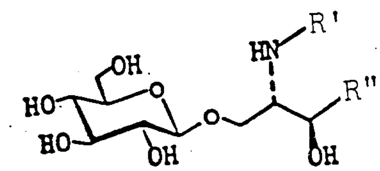

in welcher R' den Acylrest einer Fettsäure mit 14 bis 24 Kohlenstoffatomen und R" den Pentadecanyl- oder Heptadecanylrest $C_{15}H_{31}$ bzw. $C_{17}H_{35}$ oder entsprechende ungesättigte Reste bedeuten (europäische Patentanmeldung Nr. 84 11 4415.7, Veröffentlichungs-Nr. 146 810). Sie werden hergestellt durch eine Totalsynthese, ausgehend von racemischen oder optisch aktiven Ceramiden.

Bei Verwendung eines racemischen Ausgangsproduktes muss auf einer bestimmten Verfahrensstufe eine Trennung der entstandenen Diastereomeren vorgenommen werden. Die optisch aktiven Ceramide ihrerseits werden aus entsprechenden Sphingosinen erhalten, die nur auf langwierigem und ausbeutemässig unbefreidigendem Weg zugänglich sind.

Dieselben Glucosylceramide können auch aus der im Handel erhältlichen D-Galactose oder aus D- oder L-Xylose durch ein anderes Syntheseverfahren, mit besserer Ausbeute und ohne Trennung von Diastereomeren hergestellt werden (europäische Patentanmeldung Nr. 86 11 0694.6, Veröffentlichungs-Nr. 212 400).

In gleicher Weise gelang die Synthese eines Lactosylceramids mit einem Palmitoylrest als Acylrest; über diese Verbindung, insbesondere über allfällige pharmakologische Eigenschaften, ist nichts weiteres bekannt [R.R. Schmidt et al., Angew. Chem. 98 (1986), 722-723].

Das eben erwähnte und weitere Lactosylceramide, welche am Stickstoffatom jeweils einen gesättigten Acylrest tragen, sind übrigens schon früher [D. Shapiro et al., Nature 201 (1964), 878-879; Chem. Phys. Lipids 1 (1966), 54-62] hergestellt, pharmakologisch jedoch nicht näher untersucht worden; die Synthese diente nämlich als Bestätigung der einem Naturprodukt zugeteilten Strukturformel.

Aus EP-A-133 170 ist hingegen eine pharmazeutische Zusammensetzung zur Behandlung von durch Bakterien verursachten Erkrankungen bekannt, welche Lactose oder ein Lactosederivat, beispielsweise Lactosylceramid, enthält. Ferner ist aus WO-A-8 103 175 ein pharmazeutisches Präparat zur Behandlung bakterieller Harnwegsinfektionen bekannt, welches Globotetraosylceramid, Globotriaosylceramid oder Globotetraosylceramid enthält; Zusatz von Lactosylceramid, welches selbst die bakterielle Haftung nicht zu verhindern vermag, erhöht die Wirkung der Wirkstoffe.

Die Galactosylceramide und die Glucosylceramide zeichnen sich allesamt durch wundheilungsfördernde bzw. zell- und geweberegenerierende Wirkung aus und können zur Behandlung von Wunden jeglicher Genese therapeutisch verwendet werden.

Es wurde nun gefunden, dass Lactosylverbindungen der Formel I (auf beiliegendem Formelblatt) sich in ihrem pharmakologischen Verhalten wider Erwarten von dem oben beschriebenen Wirkungsmuster deutlich unterscheiden, indem sie nicht primär auf geschädigte Zellen und Gewebe heilungsfördernd und regenerierend wirken, sondern vielmehr eine spezifische zytoprotektive Wirkung gegenüber schädigenden entzündlichen Einwirkungen ausüben.

Gegenstand der Erfindung ist also die Verwendung einer Lactosylverbindung der allgemeinen Formel I zur Herstellung eines pharmazeutischen Präparates, welches als zytoprotektives Mittel, insbesondere zur Prophylaxe von Zell- und Gewebeschädigungen nicht traumatischer Herkunft verwendet werden soll. In der Formel I bedeutet $R^1$ einen aliphatischen Rest mit 9 bis 19 Kohlenstoffatomen in gerader Kette, welche eine oder mehrere Doppelbindungen oder/und seitliche Methylgruppen tragen kann, $R^2$ den Acylrest einer gesättigten oder einer einfach oder mehrfach ungesättigten Fettsäure mit 14 bis 24 Kohlenstoffatomen und X eine Gruppe der Formel $-CH_2-CH_2-$ oder $-CH=CH-$ .

Einen weiteren Gegenstand der Erfindung bilden die neuen Lactosylverbindungen der allgemeinen Formel IA, in welcher $R^1$ und X dieselbe Bedeutung wie in der Formel I haben und $R^{2'}$ den Acylrest einer einfach oder mehrfach ungesättigten Fettsäure mit 14 bis 24 Kohlenstoffatomen darstellt.

Die Lactosylverbindungen sind Abkömmlinge des Sphingosins und gehören je nach stereochemischer Konfiguration der zugrundeliegenden Sphingosinmolekel der erythro- oder der threo-Reihe an. Bezüglich

ihrer Stereochemie entsprechen die erythro-Verbindungen den bereits bekannten, in der Natur vorkommenden neutralen Glykosphingolipiden. Zur Veranschaulichung der stereochemischen Verhältnisse sei auf die vier möglichen Konfigurationen des Sphingosins verwiesen:

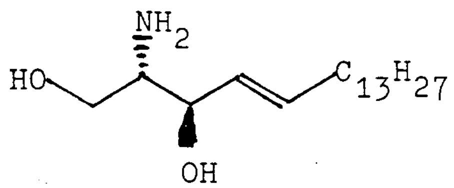

D-erythro-Sphingosin

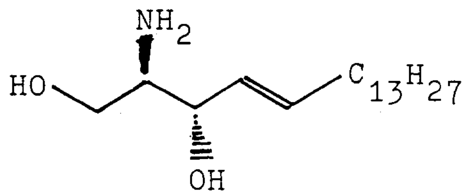

L-erythro-Sphingosin

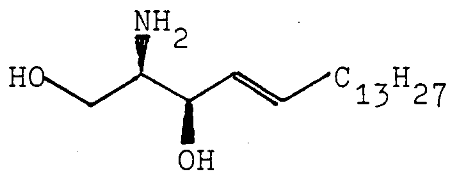

D-threo-Sphingosin

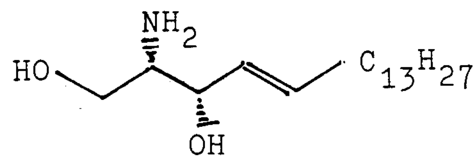

L-threo-Sphingosin

Unter den aliphatischen Ketten $R^1$ werden jene mit 13 bis 15 Kohlenstoffatomen bevorzugt. Gegebenenfalls tragen diese Ketten eine oder mehrere Doppelbindungen, z.B. eine solche in der 3,4-Stellung oder Doppelbindungen in den 3,4- und 7,8-Stellungen. Auch kann die aliphatische Kette eine oder mehrere, etwa bis 4, seitliche Methylgruppen tragen, z.B. in der 4-Stellung oder am endständigen Kohlenstoffatom.

Beispiele der gesättigten Acylreste $R^2$ sind die Acylreste der Myristinsäure $C_{14}H_{28}O_2$, der Palmitinsäure $C_{16}H_{32}O_2$, der Stearinsäure $C_{18}H_{36}O_2$ und der Arachinsäure $C_{20}H_{40}O_2$. Als Beispiele der ungesättigten Acylreste $R^2$ bzw. $R^{2'}$ seien genannt die Acylreste der Palmitoleinsäure $C_{16}H_{30}O_2$ (cis-9-Hexadecensäure), der Oelsäure und der Elaidinsäure $C_{18}H_{34}O_2$ (cis-9- bzw. trans-9-Octadecensäure), der Linolsäure $C_{18}H_{32}O_2$ (cis,cis-9,12-Octadecadiensäure), der Linolensäure und der Elaeostearinsäure $C_{18}H_{30}O_2$ - (9,12,15- bzw. 9,11,13-Octadecatriensäure), der Arachidonsäure $C_{20}H_{32}O_2$ (5,8,11,14-Eicosatetraensäure), der Eicosapentaensäure $C_{20}H_{30}O_2$, der Erucasäure und der Brassidinsäure $C_{22}H_{42}O_2$ (cis-13- bzw. trans-13-Docosensäure) und der Nervonsäure $C_{24}H_{46}O_2$ (cis-15-Tetracosensäure). Bevorzugte Acylreste sind jene mit gerader Anzahl Kohlenstoffatome in der Kette, insbesondere die $C_{16}$-, $C_{18}$- und $C_{20}$-Reste.

Ganz allgemein werden jene Präparate bevorzugt, in deren Wirkstoff der Formel I der Acylrest $R^2$ jenen der Palmitinsäure, der Oelsäure, der Linolsäure, der Linolensäure, der Arachidonsäure und der Eicosapentaensäure darstellt und der aliphatische Rest $R^1$ 13 bis 15 Kohlenstoffatome in gerader Kette umfasst.

Zur Prüfung auf eine allfällige, therapeutisch günstige Wirkung auf geschädigte Zellen und Gewebe können verschiedene pharmakologische Versuche herangezogen werden. Eine heilungsfördernde Wirkung kann insbesondere mittels des sogenannten Hautlappen-testes abgeschätzt und gemessen werden; der Test beruht auf der Bildung einer Hautnekrose bzw. deren Hemmung als Folge der Wirkung der Testsubstanz.

Es werden männliche Wistar-Ratten von 220-240 g Körpergewicht in Gruppen von 8 Tieren für jeden zu bestimmenden Wert einer Anästhesie mit Nembutal (50 mg/kg, i.p.) unterzogen. Mit einem Skalpell wird auf dem Rücken ein Hautlappen von 1 x 3 cm ausgeschnitten, ausgewaschen, an Ort und Stelle wieder eingefügt und genäht. Den Versuchstieren wird die zu prüfende Verbindung in 5 %iger Konzentration in wässriger Lösung (enthaltend 0,2 % Ethanol und 1 % Polyethylenglykol vom Molekulargewicht 20.000 - oder Carbowax® 20; jeweils 1,5 ml intraperitoneal) täglich, während 8 Tagen verabreicht; die Kontrolltiere erhalten nur das wässrige Medium. Täglich werden die Hautlappen im Massstabe 1:1 abgezeichnet und die nekrotischen Stellen dabei schwarz angefärbt. Nach 8 Tagen werden die Tiere getötet, die Zeichnungen mit einem Biotran-Apparat ausgewertet und die Gesamtfläche der nekrotischen Stellen in $mm^2$ gemessen. Die heilungsfördernde Wirkung wird durch die Zunahme der Fläche der überlebenden Haut in % gegenüber

der Kontrolle ausgedrückt.

In der vorliegenden Beschreibung haben die Abkürzungen folgende Bedeutungen:

| | | | | | |
|---|---|---|---|---|---|
| Glu : D-Glucosyl | Pal | : | Acylrest | der | Palmitinsäure |
| Gal : D-Galactosyl | Oel | : | " | " | Oelsäure |
| Lac : D-Lactosyl | Linol | : | " | " | Linolsäure |
| | Linolen | : | " | " | Linolensäure |
| | Arach | : | " | " | Arachidonsäure |

Sph : Grundkörper des $C_{18}$-Sphingosins

Tabelle 1

Heilungsfördernde Wirkung im Hautlappen-Test

| Verbindung | Fläche des Hautlappens in $mm^2$ | Fläche der nekrotischen Haut in $mm^2$ | Fläche der nekrotischen Haut in % | Fläche der überlebenden Haut in $mm^2$ | Fläche der überlebenden Haut in % | Wirkung der Verbindung in % |
|---|---|---|---|---|---|---|
| Glu(Pal)-D-Sph | 257,4 ± 6,2 | 113,3 ± 10,6 | 43,8 | 144,1 ± 9,1 | 56,2 | + 30,1 % |
| Glu(Pal)-L-Sph | 264,4 ± 2,6 | 85,8 ± 14,7 | 32,7 | 178,6 ± 16,8 | 67,3 | + 55,8 % |
| Kontrolle | 259,5 ± 4,9 | 146,6 ± 17,3 | 56,8 | 112,9 ± 19,1 | 43,2 | —— |
| Glu(Linolen)-D-Sph | 291,5 ± 5,5 | 184,1 ± 28,9 | 62,8 | 107,4 ± 27,3 | 37,2 | + 144,7 % |
| Kontrolle | 301,0 ± 4,1 | 255,5 ± 14,5 | 84,8 | 45,5 ± 12,7 | 15,2 | —— |
| Gal(Pal)-D-Sph | 273,3 ± 5,2 | 81,4 ± 18,5 | 29,8 | 191,8 ± 18,9 | 70,2 | + 31,2 % |
| Gal(Pal)-L-Sph | 281,3 ± 7,6 | 84,5 ± 13,6 | 30,1 | 196,8 ± 15,2 | 69,9 | + 30,7 % |
| Kontrolle | 267,9 ± 5,4 | 125,0 ± 27,3 | 46,5 | 142,9 ± 26,6 | 53,5 | —— |
| Lac(Pal)-D-Sph | 310,9 ± 6,9 | 113,2 ± 13,0 | 36,2 | 197,6 ± 12,6 | 63,8 | + 47,3 % |
| Lac(Pal)-L-Sph | 295,5 ± 6,4 | 112,7 ± 13,3 | 37,7 | 182,8 ± 11,3 | 62,3 | + 43,9 % |
| Kontrolle | 301,3 ± 10,9 | 172,6 ± 27,4 | 56,7 | 128,7 ± 22,5 | 43,3 | —— |
| Lac(Linolen)-D-Sph | 324,0 ± 10,0 | 128,1 ± 34,2 | 39,6 | 195,9 ± 35,5 | 60,4 | + 22,0 % |
| Kontrolle | 324,6 ± 8,8 | 164,3 ± 22,2 | 50,5 | 160,3 ± 20,6 | 49,5 | —— |
| Lac(Arach)-D-Sph | 323,1 ± 10,4 | 111,5 ± 25,8 | 35,8 | 211,6 ± 33,3 | 64,1 | + 36,8 % |
| Kontrolle | 302,6 ± 8,6 | 158,5 ± 19,3 | 52,8 | 144,1 ± 20,9 | 47,1 | —— |

Die oben wiedergegebenen Ergebnisse Zeigen für die untersuchten Verbindungen eine deutliche, für die meisten sogar eine ausgeprägte heilungsfördernde Wirkung, und zwar sowohl in der Gruppe der Lactosylceramide als auch in der zum Vergleich herangezogenen Gruppe der Glucosyl- und Galactosylceramide.

Um einen tieferen Einblick in die pharmakologischen Eigenschaften der Verbindungen der Formel I zu gewinnen, sind sie dann auf allfällige zytoprotektive Wirkungen geprüft worden. Die zytoprotektive Wirkung kann u.a. nach der von A. Wendel und G. Tiegs in Biochemical Pharmacology 35 (1986), 2115 - 2118 beschriebenen Methode gezeigt werden. Sie beruht auf der Schädigung der Leberfunktion als Folge einer

Sensibilisierung der Leber durch D-Galactosaminverabreichung und der gleichzeitigen Behandlung mit Endotoxinen.

D-Galactosamin (abgekürzt in GalN) erzeugt am Tier Leberschäden, die als Modell für humane Virus-Hepatitis gelten. Als Mechanismus wird eine Anhäufung toxischer Galactosaminderivate in der Leber angenommen, die zu einer Verminderung der Biosynthese von Makromolekülen wie Ribonucleinsäuren, Proteine, Glycoproteine und Glykogene führt. Diese metabolischen Veränderungen wiederum können in der Leber zu Zellschädigungen bis zum Zelltod führen. Das Ausmass der Leberschädigung kann an der Erhöhung von gewissen Leberenzymen (Sorbitol-Dehydrogenase SDH, Serum-Glutamatoxalat-Transaminase SGOT, Serum-Glutamatpyruvat-Transaminase SGPT) im Blut und mittels Leberhistologie bestimmt werden.

Bei gleichzeitiger Verabreichung von Lipopolysacchariden (LPS, Endotoxine gram-negativer Bakterien) und des D-Galactosamins in einer Dosierung, die an sich noch keine Erhöhung der Transaminasen (subtoxische Dosis) bewirkt, kann die letale Wirkung der LPS um einen Faktor von bis zu 100'000 gesteigert werden. Die Mechanismen der Galactosamin-bewirkten Sensibilisierung für die LPS-Wirkung sind nicht bekannt. Aus jüngsten Untersuchungen darf jedoch angenommen werden, dass die Endotoxine eine Reihe von endogenen Mediatoren (Leukotriene, tumor necrosis factor, Prostaglandine, lysosomale Enzyme, usw.) aus Makrophagen freisetzen und diese auf vorgeschädigte Hepatozyten pathogen wirken.

Diese Befunde sind insofern klinisch von Interesse, als sie zeigen, dass eine an sich harmlose Endotoxämie bei Leberschädigung (z.B. alkoholische Leberzirrhose) einen letalen Ausgang nehmen kann.

Man wusste auch bereits, dass gewisse Substanzen gegenüber der erwähnten Leberschädigung eine protektive Wirkung ausüben, wenn sie zuvor oder kurz danach dem Versuchstier verabreicht werden. Diese zytoprotektive Wirkung konnte unter anderem mit Antiphlogistica, Leukotrien-Antagonisten, Lipoxygenase-Hemmstoffen und Calcium-Calmodulin-Antagonisten erzielt werden, wie durch G. Tiegs und A. Wendel in Biochemical Pharmacology 1988 (im Druck) dargelegt und in der folgenden Tabelle 2 am Beispiel bekannter Antiphlogistica und des Diethylcarbamazins veranschaulicht wird.

Tabelle 2

| Wirkung von Antiphlogistica auf die GalN/Endotoxin-Hepatitis (aus A. Wendel, loc. cit.) | | | |
|---|---|---|---|
| Art der Behandlung | Anzahl Versuchstiere | SGOT | SGPT |
| Keine Behandlung (Basiswerte) | 10 | 90 ± 10 | 70 ± 30 |
| Kontrolle (GalN/Endotoxin) | 54 | 5580 ± 5120 | 10440 ± 8640 |
| Indomethacin 9 mg/kg | 8 | 480 ± 475* | 730 ± 1040* |
| Diethylcarbamazin 78 mg/kg, i.p. 1) | 10 | 270 ± 90** | 290 ± 180** |
| Dexamethason 200 $\mu$g/kg, i.p. | 8 | 140 ± 100* | 100 ± 60** |
| Signifikanz: | | | |

* p ≦ 0,01
** p ≦ 0,001
1) alle 45 Minuten, zwischen 0 und 6 Stunden

Allgemein dürften Substanzen in dieser Hinsicht wirksam sein, welche die Bildung oder die Freisetzung von endogenen Mediatoren aus Makrophagen hemmen, das reticuloendotheliale System aktivieren oder die Ansprechbarkeit geschädigter Zellen auf diese Mediatoren (zytoprotektive Wirkung im engeren Sinn) verhindern.

Ueberraschenderweise trifft dies für die Lactosylderivate der Formel I zu, nicht aber für die zum Vergleich ebenfalls geprüften Glucosyl- und Galactosylderivate, wie aus der nachfolgenden Tabelle 3 hervorgeht. Der Test wird folgendermassen ausgeführt.

Männliche Albinomäuse vom NMRI-Stamm werden mit 200 $\mu$g/kg Testsubstanz intraperitoneal vorbehandelt und 1 Stunde danach mit 700 mg/kg D-Galactosamin und 33 $\mu$g/kg Endotoxine intraperitoneal behandelt. 9 Stunden später wird Blut durch Herzpunktion entnommen und die enzymatische Aktivität des Blutserums nach H.U. Bergmeyer [Methods in Enzymatlc Analysis, 3. Auflage (1985), Band III] bestimmt. In diesem Versuchsmodell fungieren die Hepatozyten (Kupffer-Zellen) eigentlich als immobilisierte Makrophagen.

Der Serumgehalt an den Leberenzymen SDH, SGOT und SGPT wird in Internationalen Einheiten für die Enzymaktivität (IE) ausgedrückt; 1 IE = 1 $\mu$Mol Substratumsatz/Minute im Liter.

### Tabelle 3

Wirkung der Verbindungen I auf die GalN/Endotoxin-Hepatitis

| Art der Behandlung | Anzahl Versuchstiere | SDH in IE/Liter | SGOT in IE/Liter | SGPT in IE/Liter |
|---|---|---|---|---|
| Keine Behandlung (Basiswerte) | 6 | 40 ± 10 | 75 ± 40 | 40 ± 10 |
| Kontrolle (GalN/Endotoxin) | 14 | 3290 ± 3150 | 1640 ± 1240 | 4230 ± 4120 |
| Glu(Pal)-D-Sph | 8 | 4720 ± 2320 n.s. | 910 ± 425 n.s. | 3950 ± 2260 n.s. |
| Glu(Pal)-L-Sph | 8 | 1690 ± 1680 n.s. | 410 ± 360 n.s. | 1470 ± 1460 n.s. |
| Glu(Linolen)-D-Sph | 8 | 2280 ± 355 n.s. | 940 ± 170 n.s. | 3350 ± 630 n.s. |
| Gal(Pal)-D-Sph | 8 | 2400 ± 2800 n.s. | 1030 ± 1150 n.s. | 2990 ± 3510 n.s. |
| Gal(Pal)-L-Sph | 8 | 4610 ± 6070 n.s. | 1410 ± 1900 n.s. | 4730 ± 6210 n.s. |
| Lac(Pal)-D-Sph | 6 | 80 ± 30 * | 80 ± 20 ** | 110 ± 70 * |
| Lac(Pal)-L-Sph | 7 | 80 ± 20 * | 120 ± 80 ** | 80 ± 20 * |
| Lac(Linolen)-D-Sph | 7 | 290 ± 250 ** | 124 ± 55 * | 230 ± 76 * |
| Lac(Arach)-D-Sph | 6 | 355 ± 425 * | 280 ± 260 * | 290 ± 210 * |

Signifikanz : n.s. = nicht signifikant
* = $p \leq 0,05$
** = $p \leq 0,01$

Die protektive Wirkung der Lactosylverbindungen gegenüber der erwähnten Leberschädigung geht aus dem Vergleich der normalen SDH-, SGOT- und SGPT-Werte (keine Behandlung) mit jenen nach der Behandlung mit Galactosamin/Endotoxin und mit bzw. ohne Vorbehandlung mit der Testsubstanz eindrücklich hervor; diese Wirkung ist signifikant.

Die hervorragende Wirksamkeit der Lactosylverbindungen als zytoprotektive Mittel war umso überraschender, als sich in diesem Test die Glucosyl- und Galactosylverbindungen wirkungslos erwiesen haben : von der gleichartigen, positiven Wirkung aller genannten Verbindungen im Hautlappen-Test (siehe oben Tabelle 1) durfte man auch hier ein ähnliches pharmakologisches Verhalten erwarten.

Die spezifische zytoprotektive Wirkung der Lactosylverbindungen zeigt sich insbesondere gegenüber schädigenden entzündlichen Einwirkungen auf die Endothelzellen. Diese Wirkung erschöpft sich jedoch nicht darin, dem Einsetzen von entzündlichen Vorgängen im Organismus als Folge von Schädigungen wirksam vorzubeugen; darüber hinaus werden überschiessende pathologische Reaktionen, welche eine bereits eingetretene primäre Entzündung in einen chronischen Zustand übergehen lassen, unterdrückt. Die zytoprotektiven Eigenschaften entfalten sich also zugleich kurativ, gegenüber der primären Entzündung, und prophylaktisch, gegen das Fortschreiten und Chronischwerden der pathologischen Vorgänge.

Die erfindungsgemäss hergestellten Präparate eignen sich somit zur Anwendung als zytoprotektive Mittel, insbesondere zum Schutz der Endothelzellen gegen schädigende entzündliche Einwirkungen. Es fallen nicht darunter, wohlverstanden, unter anderem die Schnittwunden, Prellungen, Quetschungen sowie andere ähnliche traumatische Verletzungen.

Als therapeutische Indikationen gelten einerseit akute und chronische Entzündungen und andererseits Organischämien, die durch Gefässverengung bedingt sind, wie der apoplektische Insult, der Myokardinfarkt und der renale Infarkt. Beispiele der genannten Entzündungen sind die Hepatitis, die Polyarthritis, die rheumatoide Arthritis, die Myokarditis, entzündliche Hauterkrankungen, die Psoriasis, der Lupus erythematodes.

Zur prophylaktischen und kurativen Behandlung bei den erwähnten Indikationen eignet sich für den erwachsenen Menschen ein Dosisbereich von etwa 50 ng/kg bis 10 mg/kg Körpergewicht. Bei einem Menschen von 70 kg wird man als Einheitsdosis vorzugsweise eine Menge von etwa 1 µg bis 50 mg verabreichen. Die Verabreichung kann mit Vorteil parenteral, insbesondere intramuskulär und intravenös, oder topisch erfolgen. Entsprechende Darreichungsformen sind die Injektionslösungen, die Salben und die Gele.

Bekanntlich erfordern die oben angeführten Indikationen eine lang anhaltende Behandlung, bis sich ein therapeutischer Erfolg einstellt; bei prophylaktischer Anwendung der erfindungsgemäss hergestellten Präparate ist eine Dauerbehandlung die Regel. Es ist deshalb von ausschlaggebender Bedeutung, dass die Verbindungen der Formel I in einer Dosis, welche 100-mal höher als die wirksame Dosis ist, noch keinerlei toxische Symptome oder Nebenwirkungen bewirken.

Dies geht insbesondere aus einer Prüfung der Verbindung Lac(Pal)-L-Sph - genaue chemische Bezeichnung:
(2R,3S,4E)-1-[4-O-(β-D-Galactopyranosyl)-β-D-glucopyranosyloxy]-2-hexadecanoylamino-4-octadecen-3-ol - auf akute Toxizität hervor. Es wurden zwei Gruppen von je zehn Mäusen während 10 Tagen täglich beobachtet und gewogen. In der Kontrollgruppe werden den Tieren jeweils 500 µl einer 5 mM Lösung von Pluronic® F 68 (Hersteller: BASF Wyandotte, Erbslöh/BRD) in physiologischer Kochsalzlösung intraperitoneal verabreicht, in der Testgruppe hingegen 500 µl einer Lösung von 6 mg der oben bezeichneten Verbindung in 6 ml Pluronic® F 68. Die Gewichtsentwicklung in den beiden Gruppen wird durch folgende Tabelle veranschaulicht.

Tabelle 4

| Tier-Nr. | Gewicht am 10.5.88 | Gewicht am 20.5.88 | Differenz (in g) | |
|---|---|---|---|---|
| 1. | 26,8 | 32,4 | + 5,6 | Kontroll-gruppe |
| 2. | 27,7 | 34,1 | + 6,4 | |
| 3. | 29,4 | 34,2 | + 4,8 | |
| 4. | 27,5 | 33,2 | + 5,7 | |
| 5. | 30,0 | 35,7 | + 5,7 | |
| 6. | 27,0 | 32,1 | + 5,1 | |
| 7. | 30,4 | 38,1 | + 7,7 | |
| 8. | 29,5 | 33,3 | + 3,8 | |
| 9. | 27,3 | 36,6 | + 9,3 | |
| 10. | 31,2 | 37,2 | + 6,0 | |
| 1. | 29,1 | 35,7 | + 6,6 | Test-gruppe |
| 2. | 27,0 | 33,0 | + 6,0 | |
| 3. | 27,7 | 35,0 | + 7,3 | |
| 4. | 27,9 | 34,9 | + 7,0 | |
| 5. | 26,7 | 34,4 | + 7,7 | |
| 6. | 30,2 | 39,9 | + 9,7 | |
| 7. | 24,5 | 28,8 | + 4,3 | |
| 8. | 27,6 | 34,0 | + 6,4 | |
| 9. | 26,6 | 30,5 | + 3,9 | |
| 10. | 26,3 | 31,9 | + 5,6 | |

Es konnten während der Versuchsdauer und der darauf folgenden Tage keine Mortalität und keine toxischen Symptome sowie auch keine besonderen Reaktionen unmittelbar nach der Injektion beobachtet werden; die Gewichtszunahme hat sich in den beiden Tiergruppen streng vergleichbar verhalten. Die verabreichte Dosis betrug ca. 20 mg/kg Maus, d.h. 100-mal mehr als die wirksame Dosis (200 µg/kg) im Galactosamin/Endotoxin-Test (siehe weiter oben).

Einen weiteren Gegenstand der Erfindung bildet das Verfahren zur Herstellung der neuen Lactosylverbindungen der Formel IA (siehe Formelblatt).

Das Verfahren besteht darin, dass man eine Azidoverbindung der Formel II, in welcher R Wasserstoff oder eine Schutzgruppe bedeutet und $R^1$ und X obige Bedeutungen haben, mit dem O-Trifluor- oder O-Trichlor-acetimidat oder dem 1-Halogen- oder 1-Thioderivat einer Lactose, deren Hydroxylgruppen in den 2,3,6,2',3',4',6'-Stellungen durch Acylgruppen Ac geschützt sind, umsetzt, von der erhaltenen Verbindung der Formel III die Acylgruppen Ac und, wenn vorhanden, die Schutzgruppe R abspaltet, in der erhaltenen Verbindung der Formel IV die Azidogruppe in eine primäre Aminogruppe überführt und die erhaltene

Verbindung der Formel V einer N-Acylierung mit einer Fettsäure $R^{2'}$-OH oder einem reaktionsfähigen Derivat derselben unterwirft.

Das erfindungsgemässe Herstellungsverfahren wird im folgenden ausführlich erläutert.

Als Ausgangsprodukt werden Azidoverbindungen II verwendet, deren Hydroxylgruppe in 3-Stellung frei oder in geschützter Form vorliegt. Als Schutzgruppe R eignen sich vor allem einfache, aliphatische Carbonsäuren und aromatische, insbesondere monocyclische aromatische Carbonsäuren; bevorzugt wird die Verwendung der Benzoesäure, einer substituierten Benzoesäure oder der Pivalinsäure. Hergestellt werden die Ausgangsprodukte II der erythro-Reihe nach dem im EP 212 400 beschriebenen Verfahren.

Die Umsetzung der Verbindung II mit dem O-Trichlor- oder O-Trifluor-acetimidat einer Lactose, deren Hydroxylgruppen ausser jener an der 1-Stellung durch Acylreste Ac geschützt sind, wird mit Vorteil durch eine Lewis-Säure wie Bortrifluorid-etherat oder Trifluormethansulfonsäuretrimethylsilylester katalysiert. Sie wird im allgemeinen in einem wasserfreien organischen Lösungsmittel wie ein Kohlenwasserstoff (Hexan) oder ein halogenierter Kohlenwasserstoff (Dichlormethan) durchgeführt. Als Acylreste zum Schutz der Hydroxylgruppen in den 2,3,6,2',3',4',6'-Stellungen der Lactose werden vorzugsweise niedere aliphatische Acylgruppen wie die Acetyl-, Propionyl-, Pivaloyl-, Trifluoracetyl- oder Methansulfonylgruppe verwendet. Einzelheiten über die Herstellung des Reagens können der Abhandlung von R.R. Schmidt und M. Stumpp (Liebigs Ann. Chem. 1983, 1249-1256) und R.R. Schmidt, J. Michel und M. Roos (Liebigs Ann. Chem. 1984, 1343-1357) entnommen werden.

Die entsprechende Umsetzung mit dem 1-Halogenderivat der acylierten Lactose, beispielsweise mit dem Chlorid oder Bromid, wird in der Regel in Gegenwart einer Schwermetallverbindung wie Silberoxid, eines Schwermetallsalzes, wie Silbercarbonat oder Quecksilbercyanid, oder einer organischen Base, welche als säurebindende Mittel fungieren, durchgeführt (Ullmanns Encyklopädie der technischen Chemie, 4. Auflage, Band 24, Seite 757, Verlag Chemie GmbH, Weinheim BRD 1983). Die Umsetzung mit dem 1-Thioderivat erfolgt mit Vorteil in Gegenwart einer Säure.

Die Abspaltung der Acylreste Ac und der Schutzgruppe R aus der Verbindung III wird im allgemeinen durch Basen katalysiert; besonders zweckmässig dafür ist die Verwendung von Natriummethanolat in wasserfreiem Methanol bei Raumtemperatur.

In der vorletzten Verfahrensstufe wird die Ueberführung der Azidogruppe in die primäre Aminogruppe am besten durch Behandlung der Verbindung IV mit Schwefelwasserstoff bei Raumtemperatur bewerkstelligt. Hierfür wird die Verbindung beispielsweise in einem Gemisch (1:1) von Wasser und Pyridin aufgelöst. Dieselbe Ueberführung kann auch durch Hydrierung mit Natriumborhydrid oder einem anderen Reduktionsmittel, wie z.B. Natriumcyanoborhydrid, durchgeführt werden.

Die N-Acylierung der Verbindung V mit der organischen Carbonsäure der Formel $R^{2'}$-OH (letzte Verfahrensstufe) kann nach der Methode von D. Shapiro und Mitarbeiter [J. Am. Chem. Soc. 86, 4472 (1964)] durchgeführt werden. Im allgemeinen wird man die Carbonsäure selbst in Gegenwart eines wasserabspaltenden Mittels, wie Dicyclohexylcarbodiimid in Dichlormethan, oder ein funktionelles reaktionsfähiges Derivat der Carbonsäure, wie einen aktivierten Ester oder ein Halogenid in Gegenwart einer anorganischen Base wie Natriumacetat oder einer tertiären organischen Base, einsetzen. Die N-Acylierung wird mit Vorteil bei Raumtemperatur durchgeführt.

Die Isolierung und Reinigung der bei jeder Verfahrensstufe anfallenden Verbindungen erfolgt nach den üblichen Methoden der organischen Chemie.

Die folgenden Beispiele veranschaulichen bevorzugte Ausführungsformen der Erfindung.

$^1$H-NMR-Spektren wurden mit dem 250 MHz-Gerät WM 250 Cryospec der Firma Bruker, Spectrospin, Industriestrasse 26; CH-8117 Fällanden/Zürich, gemessen. Die Verschiebungen sind auf Tetramethylsilan (TMS) als internen Standard bezogen und in ppm angegeben.

Die angegebenen Schmelzpunkte wurden auf einem Kupferblock bestimmt und sind nicht korrigiert.

Zur analytischen Dünnschichtchromatographie (DC) wurden Kieselgelplatten der Firma E. Merck AG, Darmstadt (BRD), verwendet. Die Dünnschichtchromatogramme wurden, sofern die Substanzen nicht UV-aktiv waren, mit 15% Schwefelsäure besprüht und bei 120 °C entwickelt.

Präparative Säulenchromatographien wurden mit Kieselgel 60(0,062-0,200 mm) der Firma Merck durchgeführt. Für die Mitteldruckchromatographie wurden Fertigsäulen mit Kieselgel "LiChroprep Si 60,15-25" verwendet.

Die Ausbeuten wurden auf der Reinigungsstufe angegeben, auf der NMR-spektroskopisch und mittels Dünnschichtchromatographie keine Verunreinigungen nachzuweisen waren.

Bei den Lösungsmittelgemischen bedeutet die Angabe in Klammern Volumenteile.

Herstellung der Ausgangsprodukte

Das (2,3,6,2',3',4',6'-Hepta-O-acety1-α-D-lactosyl)-trichloracetimidat - Verbindung (1) - wird nach Liebigs Ann. Chem. 1984, 1343-1357 hergestellt.

Das (2S,3R)-2-Azido-3-benzoyloxy-1-hydroxy-4-trans-octadecen - Verbindung (2) - wird nach EP 212 400, Seite 22 hergestellt.

Allgemeine Vorschrift zur Herstellung der Carbonsäurechloride der Formel $R^{2'}Cl$, nämlich:

9-cis-Octadecenoylchlorid - Verbindung (6)

9-cis-12-cis-Octadecadienoylchlorid - Verbindung (7)

9-cis-12-cis-15-cis-Octadecatrienoylchlorid - Verbindung (8)

6 mMol der ungesättigten Carbonsäure werden mit 2,25 g (18 mMol) frisch destilliertem Oxalylchlorid bei 70 °C unter Feuchtigkeitsausschluss am Rückfluss zum Sieden erhitzt. Nach 4 Stunden Reaktionsdauer wird überschüssiges Oxalylchlorid durch Erwärmen der Lösung im Vakuum bis 70 °C entfernt.

5-cis-8-cis-11-cis-14-cis-Eicosatetraenoylchlorid - Verbindung (9)

50 mg (164 µMol) Arachidonsäure werden mit 40 mg (320 µMol) frisch destilliertem Oxalylchlorid in 3 ml wasserfreiem Benzol 4 Stunden unter $N_2$-Schutzgasatmosphäre am Rückfluss zum Sieden erhitzt. Anschliessend wird die Reaktionslösung eingeengt und bei 80 °C im Vakuum überschüssiges Oxalylchlorid entfernt.

Beispiel 1

(2S,3R)-2-(9-cis-Octadecenoylamino)-3-hydroxy-1-(β-D-lactosyloxy)-4-trans-octadecen (10)

(2S,3R)-2-Azido-3-benzoyloxy-1-(2,3,6,2',3',4',6'-hepta-O-acetyl-β-D-lactosyloxy)-4-trans-octadecen (3)

1 g (2,33 mMol) Verbindung (2) und 2,75 g (3,66 mMol) Lactosyltrichloracetimidat (1) werden in 50 ml wasserfreiem Tetrachlorkohlenstoff gelöst. Nach Zugabe von zuvor im Hochvakuum erhitztem Molekularsieb 4Å wird die Lösung einige Minuten bei Raumtemperatur kräftig gerührt. Bei sukzessiver Zugabe von insgesamt 1,1 ml einer 0,1 molaren Lösung von Bortrifluorid-etherat in Methylenchlorid über einen Zeitraum von 4 Stunden reagiert der zuerst gebildete Orthoester zum Glycosid (3) ab (Kontrolle durch Dunnschichtchromatographie, im folgenden mit DC abgekürzt). Bei der Spaltung des Orthoesters neu entstandene Verbindung (2) wird durch Zusatz einer Spatelspitze Lactosyltrichloracetimidat (1) abgefangen.

Die Reaktionslösung wird auf 150 ml Petrolether gegeben und nach Filtration des ausgefallenen Trichloracetamids mit gesättigter $NaHCO_3$-Lösung neutralisiert. Die abgetrennte organische Phase wird über Magnesiumsulfat getrocknet und im Vakuum eingeengt. Die Reinigung des Produktes erfolgt säulenchromatographisch (Kieselgel; Petrolether/Ethylacetat 65:35).
Ausbeute: 2,1 g (85 % bezogen auf Verbindung 2) farbloses Oel
DC: (Petrolether/Ethylacetat 6:4) $R_f$ = 0,46 Glycosid (3)
$R_f$ = 0,41 Orthoester
$[\alpha]_D^{20}$ = -22,3° (c = 3,8, $CHCl_3$)
$^1$H-NMR ($CDCl_3$) δ = 8,06-7,42 (m,5H,$C_6H_5$-), 5,96-5,85 (m,1H,-$CH_2$-C͟H = CH-), 5,65-5,48

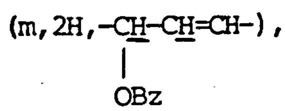

5,35-5,34 (d,1H,4'-H, J = 2,7 Hz), 5,23-5,07 (m,2H,2'-H,3-H), 4,98-4,89 (m,2H, 2H,3'-H), 4,53-4,45 (m,3H,1-H,1'-H,4-H), 4,12-4,02 (m, 3H,$CH_2O$,5'-H), 3,97-3,79 (m,4H), 3,65-3,54 (m,2H), 2,20-1,96 (m,23H,7-$CH_3$,CH = CH-$CH_2$), 1,37-1,24 (m,22H, 11-$CH_2$-), 0,90-0,85 (t,3H,$CH_3$-$CH_2$-)

| Analyse für $C_{51}H_{73}O_{20}N_3$ (Mol.gew. 1048,15) | | | | | | |
|---|---|---|---|---|---|---|
| berechnet:<br>gefunden : | C | 58,44<br>58,35 | H | 7,02<br>7,08 | N | 4,01<br>3,99 |

(2S,3R)-2-Azido-3-hydroxy-1-(β-D-lactosyloxy)-4-trans-octadecen (4)

Einer Lösung von 5,9 g (5,65 mMol) Verbindung (3) in 130 ml wasserfreiem Methanol werden 0,5 ml einer 1 molaren Natriummethanolat-Lösung in Methanol zugetropft (DC-Kontrolle). Nach 4 Stunden Rühren bei Raumtemperatur wird die Reaktionslösung mit mehrmals mit Methanol säurefrei gewaschenem Amberlite Ionenaustauscher (IR 120, $H^+$-Form) versetzt und bis zur völligen Neutralisierung gerührt. Nach Einengen der Lösung bleibt nahezu quantitativ ein farbloses Oel zurück.
Ausbeute: 3,6 g (99%)
DC: (Chloroform/Methanol 6:4) $R_f$ = 0,61
$R_f$ = 0,70 (Monoacetyl-Verbindung)

(2S,3R)-2-Amino-3-hydroxy-1-(β-D-lactosyloxy)-4-trans-octadecen (5)

3,6 g (5,6 mMol) Verbindung (4) werden in 50 ml Pyridin und 70 ml Wasser gelöst. Unter Rühren wird mit konz. Salzsäure aus Natriumhydrogensulfid entwickelter Schwefelwasserstoff 10 Min.lang eingeleitet. Nach 18 Stunden Rühren bei Raumtemperatur ist die Reaktion beendet. Die Lösung wird eingeengt, die Reinigung erfolgt chromatographisch (Chloroform/Methanol/Wasser 9:1:0→8:2:0→5:4:1).
Ausbeute: 3,28 g (95%) farblose Kristalle, bei der Schmelzpunktbestimmung konnte eine langsame Zersetzung ab 110 °C beobachtet werden.
DC: (Chloroform/Methanol/Wasser 5:4:1) $R_f$ = o,71
$[\alpha]_D^{20}$ = -8,9 ° (c = 1,2 , Pyridin)
$^1$H-NMR (DMSO-d6) 5,57 (m,1H,HC=C$\underline{H}$-$CH_2$), 5,45 (dd,1H,HC=C$\underline{H}$-CH-OH, J=15,5Hz,J= 6,7Hz), 5,12 (s,1H,OH), 4,82-4,47 (m,6H,6OH), 4,25-4,15 (m,2H), 3,86-2,77 (m,21H, 14 Zuckerprotonen, -O-$CH_2$-,C$\underline{H}$-N$\underline{H}_2$,HC=CH-C$\underline{H}$-O$\underline{H}$), 2,01-1,95 (m,2H,HC=CH-C$\underline{H}_2$-), 1,33-1,20 (m,22H, 11-$CH_2$-), 0,86 (t,3H,$CH_3$-)

(2S,3R)-2-(9-cis-Octadecenoylamino)-3-hydroxy-1-(β-D-lactosyloxy)-4-trans-octadecen (10)

Nach der Säurechlorid-Methode, aus den Verbindungen (5) und (6): 310 mg (500 µMol Verbindung (5) werden in 35 ml Tetrahydrofuran fein suspendiert und 18 ml einer gesättigten Natriumacetatlösung zugegeben. Unter intensive Rühren werden 500 µMol des Säurechlorids (6) langsam zugetropft. Nach 10 - 30 Min. (DC-Kontrolle) ist die Umsetzung beendet. Das Reaktionsgemisch wird mit 200 ml Tetrahydrofuran verdünnt die wässrige Phase getrennt und die organische mit gesättigter NaCl-Lösung gewaschen. Nach Trocknen der Lösung über $MgSO_4$ wird sie am Wasserstrahlvakum bis zur Trockne eingeengt.
Die Reinigung erfolgt a) über Acetylierungs-/Entacetylierungsschritte (siehe Anhang, Verbindungen 16 und 17) oder b) über Umkehrphase-Mitteldruckchromatographie (n-Butanol/Methanol/Wasser 50:32:18). Das Produkt fällt als farbloser Schaum an.
Ausbeute: a) 271 mg (61%) b) 324 mg (73%)
DC: (Chloroform/Methanol 8:2) $R_f$ = 0,32
Schmelzpunkt: ab 210 ° C langsame Zersetzung
$[\alpha]_D^{20}$ = -10,2 ° (c = 1,0 , Pyridin)

Verbindung (10):

$^1$H-NMR (DMSO-d6) 7,54(d,1H,NH,J=8,8Hz), 5,57-5,49 (m,1H, HC=C$\underline{H}$-$CH_2$), 5,39-5,30 (m,3H,HC=C$\underline{H}$-CH-OH, $\underline{H}$C=C$\underline{H}$), 5,16 (d,1H OH,J=3,7Hz), 5,12 (d, 1H,OH), 4,90 (d,1H,OH,J=5,5Hz), 4,84 (d, 1H,OH,J=3,1Hz), 4,68 (m,2H,OH), 4,63-4,54 (m,2H,OH), 4,22-3,29 (m,17H), 3,09-3,02 (m,1H), 2,06-1,96 (m,8H,CO$CH_2$,HC=CH-C$\underline{H}_2$, C$\underline{H}_2$-CH=CH-C$\underline{H}_2$), 1,48-1,23 (m,44H,-$CH_2$-), 0,91-0,83 (t,6H,$CH_3$-)

| Analyse für $C_{48}H_{89}O_{13}N \cdot 1,0\ H_2O$ (Molekulargewicht 906,25) | | | | | | |
|---|---|---|---|---|---|---|
| berechnet:<br>gefunden : | C | 63,62<br>63,49 | H | 10,12<br>10,02 | N | 1,55<br>1,81 |

Genaue chemische Bezeichnung:
(2S,3R,4E)-1-[4-O-(β-D-Galactopyranosyl)-β-D-glucopyranosyloxy]-2-[(9Z)-9-octadecenoylamino]-4-octadecen-3-ol

Beispiel 2

(2S,3R)-2-(9-cis-12-cis-Octadecadienoylamino)-3-hydroxy-1-(β-D-lactosyloxy)-4-trans-octadecen (11)

1. Nach der Säurechlorid-Methode, aus den Verbindungen (5) und (7); Die experimentelle Vorschrift zur Darstellung der Verbindung (11) ist analog der Umsetzung zur Verbindung (10).
Ansatz: 700 mg (1,129 mMol) Verbindung (5)
362 µl (1,140 mMol) Linolsäurechlorid (7)
50 ml Tetrahydrofuran, 25 ml wässrige gesättige Natriumacetat-Lösung.
Die Reinigung erfolgt a) über Acetylierungs-/Entacetylierungsschritte (siehe Anhang, Verbindungen 16 und 17) oder b) über Umkehrphase-Mitteldruckchromatographie (n-Butanol/Methanol/Wasser 50:32:18). Das Produkt fällt als farbloser Schaum an.
Ausbeute:

| | |
|---|---|
| a) 640 mg (64%) | b) 710 mg (71%) |

2. Nach der Methode mit 2-Ethoxy-1-ethoxycarbonyl-dihydrochinolin (EEDQ)
230 mg (371 µMol) Verbindung (5), 115 µl (371 µMol) Linolsäure und 95 mg (380 µMol) EEDQ werden in 50 ml wasserfreiem Ethanol gelöst und bei 50° C gerührt. Nach 12 Stunden wird kein Fortschreiten der Reaktion mehr beobachtet. Die Lösung wird eingeengt und das Rohprodukt an Kieselgel (Chloroform/Methanol 85:15) chromatographiert. Ausbeute: 103 mg (58%).

Charakterisierung:
farbloser Schaum vom Schmelzpunkt 138° - 145° C
DC: (Chloroform/Methanol 8:2) $R_f$ = 0,32
$[\alpha]_D^{20}$ = -8,7° (c = 1,1 , Pyridin)

Verbindung (11):

$^1$H-NMR (DMSO-d6) 7,51 (d,1H,NH,J=8,3Hz), 5,57-5,48 (m,1H, HC=CH-CH-OH), 5,39-5,24 (m,5H,HC=CH-$CH_2$, HC=CH-$CH_2$-CH=CH), 5,14 (d,1H,OH,J=4,0Hz), 5,10 (d,1H,OH,J=3,6Hz), 4,87 (d,1H,OH, J=5,5Hz), 4,79 (d,1H,OH,J=4,9), 4,68-4,66 (m,2H,OH), 4,58 (t,1H,OH,J=5,95Hz), 4,54 (d,1H,OH,J=4,6Hz), 4,21-3,29 (m,17H), 3,10-3,01 (m,1H), 2,72 (t,2H,HC=CH-$CH_2$-CH=CH), 2,09-1,92 (m,8H,CO-$CH_2$,3 HC=CH-$CH_2$), 1,44-1,23 (m,38H,-$CH_2$-), 0,88-0,83 (2 t,6H,$CH_3$-)

| Analyse für $C_{48}H_{87}O_{13}N \cdot 2,0\ H_2O$ (Mol.gew. 922,24) | | | | | | |
|---|---|---|---|---|---|---|
| berechnet:<br>gefunden : | C | 62,51<br>62,10 | H | 9,95<br>9,71 | N | 1,52<br>1,67 |

Genaue chemische Bezeichnung:
(2S,3R,4E)-1-[4-O-(β-D-Galactopyranosyl)-β-D-glucopyranosyloxy]-2-[(9Z,12Z)-9,12-octadecadlenoylamino]-4-octadecen-3-ol

Anhang: Produktreinigung über Acetylierung-/Entacetylierungsschritte

1. Acetylierung

(2S,3R)-2-(9-cis-Octadecenoylamino)-3-acetoxy-1-(2,3,6,2',3',4',6'-hepta-O-acetyl-β-D-lactosyloxy)-4-trans-octadecen (16) und (2S,3R)-2-(9-cis-12-cis-Otadecadienoylamino)-3-acetoxy-1-(2,3,6,2'3',4',6'-hepta-O-acetyl-β-D-lactosyloxy)-4-trans-octadecan (17)

400 mg der verunreinigten Cerebroside (10) und (11) werden in 4 ml wasserfreiem Acetanhydrid und 4 ml wasserfreiem Pyridin gelöst und bei Raumtemperatur gerührt. Nach 12 Stunden konnten DC-chromatographisch keine Acertylierungszwischenstufen mehr detektiert werden. Die Lösung wird am Wasserstrahlvakuum, später am Hochvakuum eingeengt und das Produkt an Kieselgel chromatographiert (Petrolether/Ethylacetat 1:1). Die Produkte fallen als farbloser fester Schaum an. DC: (Petrolether/Ethylacetat 1:1) $R_f = 0,36$
Schmelzpunkte: (16):121 °C; (17):120 °C
(16): $[\alpha]_D^{20} = -1,8°$ (c = 1,3 , Chloroform)
(17): $[\alpha]_D^{20} = -0,7°$ (c = 1,0 , Chloroform)

Verbindung (16):

$^1$H-NMR ($CDCl_3$) 5,86-5,72 (m,1H,HC=CH-$CH_2$), 5,66 (d,1H,NH,J= 9,4 Hz), 5,40-5,05 (m,7H,HC=CH-CH-OAc,HC=CH, 4'-H,3-H,2'-H), 4,95 (dd,1H,3'-H,J=3,4 Hz,J= 7,7 Hz), 4,85 (dd,1H,2-H,J=7,5 Hz,J=7,7 Hz), 4,55-4,40 (m,3H,1-H,1'-H,4-H), 4,37-4,25 (m, 1H,N-CH), 4,16-4,03 (m,3H,$CH_2$O,5'-H), 3,95-3,75 (m,3H), 3,65-3,45 (m,2H), 2,23-1,93 (m, 32H,8 Acetyl,$COCH_2$,HC=CH-$CH_2$,$CH_2$-CH=CH-$CH_2$), 1,58 (m,2H,CO-$CH_2$-$CH_2$-), 1,19 (s,38H,-$CH_2$-), 0,85 (t,6H,$CH_3$-)

| Analyse für $C_{64}H_{105}O_{21}N$ (Mol.gew. 1224,53) | | | | | | |
|---|---|---|---|---|---|---|
| berechnet:<br>gefunden : | C | 62,78<br>62,75 | H | 8,64<br>8,68 | N | 1,14<br>1,15 |

Verbindung (17):

$^1$H-NMR ($CDCl_3$) 5,84-5,72 (m,1H,HC=CH-$CH_2$), 5,65 (d,1H,NH, J=9,15 Hz), 5,40-5,07 (m,9H,HC=CH-CH-OAc,4'-H, 3H,2'-H,HC=CH-$CH_2$-CH=CH), 4,97 (dd,1H,3'-H, J=3,35 Hz,J=8,8 Hz), 4,87 (dd,1H,2-H,J=7,6 Hz, J=7,6 Hz), 4,54-4,41 (m,3H,1-H,1'-H,4-H), 4,31 (m,1H,N-CH), 4,13-4,02 (m,3H,$CH_2$O,5'-H),3,95-3,76 (m,3H), 3,63-3,49 (m,2H), 2,76 (t,1H, HC=CH-$CH_2$-CH=CH,J=6,1 Hz), 2,16-1,96 (m,32H, 8 Acetyl,3 HC=CH-$CH_2$,$COCH_2$), 1,6 (m,2H,CO-$CH_2$-$CH_2$-), 1,25 (m,34H,-$CH_2$-), 0,88 (t,6H,$CH_3$-)

| Analyse für $C_{64}H_{103}O_{21}N$ (Mol.gew. 1222,51) | | | | | | |
|---|---|---|---|---|---|---|
| berechnet:<br>gefunden : | C | 62,88<br>62,79 | H | 8,49<br>8,44 | N | 1,15<br>1,24 |

2. Entacetylierung der Verbindungen (16) und (17)

150 mg (122,6 µMol) der Verbindungen (16) und (17) in 15 ml wasserfreiem Methanol werden mit 500 mg Florisil (Magnesiumsilikat, 200-300 mesh / Fluka AG) versetzt. Das heterogene Gemisch wird 24 Stunden bei 40 °C gerührt. Nach Beendigung der Reaktion werden 10 ml Chloroform zugesetzt und vom Feststoff abfiltriert. Nach Eindampfen des Lösungsmittels im Vakuum erhält man das Produkt als farblosen Schaum.
Ausbeute: 108 mg (100%). Charakterisierung der Produkte siehe vorne.

Beispiel 3

(2S,3R)-2-(9-cis-12-cis-15-cis-Octadecatrienoylamino)-3-hydroxy-1-(β-D-lactosyloxy)-4-trans-octadecen - (12)

Nach der Säurechlorid-Methode, aus den Verbindungen (5) und (8); Die experimentelle Vorschrift zur Darstellung der Verbindung (12) ist analog der Umsetzung zur Verbindung (10).
Ansatz: 600 mg (967µMol) Verbindung (5)
307 µl (966µMol) Linolensäurechlorid (8)
50 ml Tetrahydrofuran/ 25 ml wässrige gesättigte Natriumacetat-Lösung
Die Reinigung erfolgt über Umkehrphase-Mitteldruckchromatographie (n-Butanol/Methanol/Wasser 50:32:18).
Ausbeute: 635 mg (74%) farbloser Schaum vom Schmelzpunkt 145° - 151° C
DC: (Chloroform/Methanol 8:2) $R_f$ = 0,32
$[\alpha]_D^{20}$ = -4,6° (c = 1,15 , Pyridin)

Verbindung (12):

12 $^1$H-NMR (DMSO-d6) 7,52 (d,1H,NH,J=8,9Hz), 5,60-5,48 (m,1H, HC=CH-CH-OH), 5,38-5,25 (m,7H,HC=CH-$CH_2$,3 HC=CH), 5,16 (d,1H,OH,J=3,7Hz), 5,13 (d, 1H,OH,J=3,6Hz), 4,89 (d,1H,OH,J=5,5Hz), 4,82 (d,1H,OH,J=3,7Hz), 4,67 (m,2H,OH), 4,59 (t,1H,OH,J=5,95Hz), 4,54 (d,1H,OH, J=4,6Hz), 4,21 -3,29 (m,17H), 3,07-3,06 (m,1H), 2,79-2,74 (t,4H,2 HC=CH-$CH_2$-CH=CH) 2,10-1,92 (m,8H,$COCH_2$,3 HC=CH-$CH_2$), 1,48 -1,43 (m,2H,CO-$CH_2$-$CH_2$-), 1,36-1,23 (m, 30H,-$CH_2$-), 0,93 (t,3H,HC=CH-$CH_2$-$CH_3$,J= 7,7Hz), 0,85 (t,3H,$CH_3$-,J=6,6Hz)

UV-Spektrum:

Zum Abschätzen des Anteils konjugierter Systeme werden die Extinktionen mit Standard-Absorptionswerten von konjugiert-ungesättigten Carbonsäuren verglichen [J.Amer.Chem.Soc. 66 (1944), 287-289]. In solchen Verbindungen findet man folgende Extinktionskoeffizienten [Proc.Natl.Acad. Sci. U.S. 69 (1972), 3561-3566]:

| | | |
|---|---|---|
| $\lambda_1$ = 232 nm | ($\epsilon$=33600) | Dien |
| $\lambda_2$ = 270 nm | ($\epsilon$=46700) | Trien |
| $\lambda_3$ = 320 nm | ($\epsilon$=57700) | Tetraen |

Im UV-Spektrum der Verbindung (12) wurden folgende Extinktionskoeffizienten gemessen.

| | |
|---|---|
| $\lambda_1$ = 232 nm | $\epsilon$ = 323 |
| $\lambda_2$ = 270 nm | $\epsilon$ = 70 |
| $\lambda_3$ = 320 nm | $\epsilon$ = 29 |

Daraus ergibt sich für konjugierte Diene ein maximaler prozentualer Anteil von 0,95%, für konjugierte Triene einer von 0,15% und für konjugierte Tetraene einer von 0,05% am Gesamtprodukt.

| Analyse für $C_{48}H_{85}O_{13}N \cdot 1,0, H_2O$ (Mol.gew. 902,22) | | | | | | |
|---|---|---|---|---|---|---|
| berechnet: | C | 63,90 | H | 9,72 | N | 1,55 |
| gefunden : | | 63,43 | H | 9,92 | N | 1,62 |

Genaue chemische Bezeichnung:

(2S,3R,4E)-1-[4-O-(β-D-Galactopyranosyl)-β-D-glucopyranosyloyy]-2-[(9Z,12Z,15Z)-9,12,15-octadecatrienoylamino]-4-octadecen-3-ol

Beispiel 4

(2S,3R)-2-(5-cis-8-cis-11-cis-14-cis-Eicosatetraenoylamino)-3-hydroxy-1-(β-D-lactosyloxy)-4-trans-octadecen (13)

Nach der Säurechlorid-Methode, aus den Verbindungen (5) und (9): Die Umsetzung des Säurechlorids mit der Verbindung (5) ist analog zur Darstellung der Verbindungen (10).
Ansatz: 53,1 mg Arachidonoylchlorid (9) (164µMol)
102 mg Verbindung (5) (164µMol)
10 ml Tetrahydrofuran / 5 ml wässrige gesättigte Natriumacetat-Lösung
Die Reinigung der Verbindung (13) erfolgt über Umkehrphasen-Mitteldruckchromatographie (n-Butanol/Methanol/Wasser 50:32:18).
Ausbeute: 110 mg (74%) farbloser Schaum
DC: (Chloroform/Methanol 72:28) $R_f$ = 0,49
Schmelzpunkt: von 115° bis 135°C unter Zersetzung

Verbindung (13):

$^1$H-NMR (DMSO-d6) 7,52 (d,1H,NH,J=9,0Hz), 5,55-5,49 (m,1H, HC=CH-$CH_2$), 5,39-5,32 (m,9H,HC=CH-CH-OH, 4 HC-CH), 5,13 (d,1H,OH,J=3,9Hz), 5,10 (d,1H,OH,J=4,6Hz), 4,87 (d,1H,OH,J=5,3 Hz), 4,78 (d,1H,OH,J=4,9Hz), 4,67-4,64 (d,t,2H,OH), 4,57 (t,1H,OH,J=6,0Hz), 4,19 (d,1H,1-H,J=7,1Hz), 4,15 (d,1H,1-H,J=7,8 Hz), 4,00-3,96 (m, 1H), 3,92-3,87 (m, 1H), 3,82-3,72 (m,2H), 3,63-3,61 (m,2H), 3,58-3,28 (m,9H), 3,06-3,02 (m,1H), 2,81-2,76 m,6H,3 HC=CH-$CH_2$-CH=CH), 2,09-1,97 (m,6H, 3 HC=CH-$CH_2$),1,93-1,91 (m,2H,$COCH_2$), 1,54 -1,49 (m,2H,CO-$CH_2$-$CH_2$-), 1,35-1,23 (m, 28H,$CH_2$-), 0,87-0,83 (2 t,6H,$CH_3$-)
UV-Spektrum: Das UV-Spektrum wurde analog zu jenem der Verbindung (12) ausgewertet. Der maximale Anteil konjugierter Diene beträgt 2,1%, jener konjugierter Triene 0,6% und jener konjugierter Tetraene 0,2%.

| Analyse für $C_{50}H_{87}O_{13}N \cdot 2{,}0\ H_2O$ (Mol.gew. 946,27) | | | | | | |
|---|---|---|---|---|---|---|
| berechnet:<br>gefunden : | C | 63,46<br>63,06 | H | 9,69<br>9,80 | N | 1,48<br>1,62 |

Genaue chemische Bezeichnung:

(2S,3R,4E)-1-[4-O-(β-D-Galactopyranosyl)-β-D-glucopyranosyloxy]-2-[(5Z,8Z,11Z,14Z)-5,8,11,14-icosatetraenoylamino]-4-octadecen-3-ol

Beispiel 5

(2S,3R)-2-(5-cis-8-cis-11-cis-14-cis-Eicosatetraenoylamino)-3-hydroxy-1-(β-D-lactosyloxy)-4-trans-octadecen (13)

150 mg (242 µMol) Verbindung (5) und 1,17 ml (3,63 mMol) Arachidonoylchlorid - Verbindung (9) - werden in 15 ml wasserfreiem Pyridin gelöst. Nach Zusatz einer Spatelspitze 4-Dimethylamino-pyridin wird das Reaktionsgemisch 2 Tage bei 75°C gerührt (DC-Kontrolle). Es wird zur Trockne eingeengt, über Mitteldruckchromatographie (Petrolether/Ethylacetat 92:8) gereinigt und mit Natriummethanolat behandelt. Die erhaltene Verbindung (13) weist dieselben Eigenschaften auf, die im Beispiel 4 angegeben werden.

Beispiel 6: Injektionslösung

In 100 ml physiologischer Kochsalzlösung (0,9% NaCl) werden 5 g Pluronic® F 68 und 500 mg Verbindung Lac(Pal)-D-Sph - (2S,3R,4E)-1-[4-O-(β-D-Galactopyranosyl)-β-D-glucopyranosyloxy]-2-hexadecanoylamino-4-octadecen-3-ol - unter Rühren aufgelöst, die Lösung wird durch Filtration über ein Filter Millipore 22 sterilfiltriert und in Portionen von 1 ml in Ampullen abgefüllt. Eine Ampulle enthält 5 mg des oben genannten Wirkstoffes.

Beispiel 7: Gel

a) In 50 ml demineralisiertem Wasser werden 100 mg Verbindung Lac(Oel)-D-Sph (siehe Beispiel 1) aufgelöst, der Lösung werden zuerst 3 g Diisopropylamin und dann langsam, unter Rühren, 3 g Carbopol® 940 (Polyacrylsäure von durchschnittlichem Molekulargewicht 4'000'000; Hersteller: Goodrich Chemical Co., Cleveland OH/USA) zugegeben, das Gemisch wird über Nacht stehengelassen und dann durch langsame Zugabe von demineralisiertem Wasser unter ständigem Rühren auf ein Endvolumen von 100 ml gebracht. 1 g Gel enthält 1,0 mg Wirkstoff.

b) Man löst 2,0 g Diisopropanolamin in 10 g demineralisiertem Wasser unter Bildung einer Lösung A. Andererseits mischt man 50 g Ethanol 95% und 5,0 g Diisopropyladipat als rückfettendes Mittel miteinander, löst darin unter Rühren 200 mg Verbindung Lac(Pal)-L-Sph und gibt langsam so viel der Lösung A hinzu, dass ein pH von 6,8 erreicht wird (Lösung B).

Man mischt 2 g Carbopol® 941 (Polyacrylsäure vom Mol.gew. 1'250'000) mit 20 Teilen demineralisiertem Wasser, lässt während einiger Zeit aufquellen, rührt bis zur Bildung einer homogenen Masse und gibt unter Rühren soviel der Lösung A hinzu, bis das pH auf 6,8 eingestellt ist; es entsteht ein Gel C.

Man mischt nun die Lösung B langsam und unter ständigem Rühren in das Gel C hinein, um ein einheitliches Gel zu bilden, und bringt schliesslich auf ein Endgewicht von 100 g durch Zugabe von demineralisiertem Wasser. Erhalten wird ein Gel von 2,0 mg Wirkstoff per 1 g.

Beispiel 8: Salbe

In 35 g dickflüssigem Paraffin werden 500 mg Verbindung Lac(Linol)-D-Sph (siehe Beispiel 2) suspendiert, der Suspension werden 35 g Cetylstearylalkohol und 30 g weisses Vaselin zugegeben, das Gemisch wird im Wasserbad langsam auf die Schmelztemperatur erhitzt, die geschmolzene Masse wird bei derselben Temperatur während 20 Minuten bei geringer Geschwindigkeit (etwa 60 U/Min.) gerührt, das Erhitzen wird abgestellt und die Masse bis zum Erkalten weiterhin gerührt. 1 g Salbe enthält 5 mg Wirkstoff.

Beispiel 9: Crème

Zuerst wird durch kräftiges Rühren eine homogene Mischung aus 12 g 1-Propyloxypropan-2,3-diol, 6 g 1-n-Hexyloxypropan-2,3-diol, 6 g 1-n-Nonyloxypropan-2,3-diol, 24 g Wasser und 1,0 g Phenoxyethanol hergestellt. Der Mischung wird 1,0 g Verbindung Lac(Linolen)-D-Sph (siehe Beispiel 3) zugegeben und unter Rühren aufgelöst. Andererseits werden 30 g weisses Vaselin, 15 g Cetylalkohol und 5 g Sorbitanmonopalmitat vermischt und im Wasserbad durch langsames Erwärmen auf eine Temperatur von etwa 70 °C geschmolzen. In der geschmolzenen Masse wird nun die ebenfalls auf etwa 70 °C erwärmte Wirkstofflösung unter Rühren bei hoher Tourenzahl dispergiert und unter weiterem Rühren abkühlen gelassen. Erhalten wird eine Wasser-in-Oel-Emulsion (Crème), welche in 1 g 10 mg Wirkstoff enthält.

Beispiel 10: Injektionslösung

2 g Pluronic® F 68 werden in 100 ml physiologischer Kochsalzlösung aufgelöst, der Lösung werden 2 g Verbindung Lac(Arach)-D-Sph (siehe Beispiel 4) zugegeben und durch Rühren aufgelöst, die Lösung wird durch ein Filter Millipore 22 sterilfiltriert und in Portionen von 1 ml in Ampullen abgefüllt. Eine Ampulle enthält 20 mg Wirkstoff.

Herstellung der Ausgangsprodukte

Das D-threo-Sphingosin kann nach dem Verfahren von P. Herold [Helv. Chim.Acta 71 (1988), 354-362] hergestellt werden.

Synthese des L-threo-Sphingosins

(2S,3S,4E)-2-Amino-1,3-dihydroxy-4-octadecen 2,4-O-Benzyliden-D-threose - Verbindung (1 ) und
2,4-O-Benzyliden-D-erythrose - Verbindung (14)

Herstellung eines Phosphatpuffers von pH 7,6:
10,4 g $NaH_2PO_4 \cdot 2\ H_2O$ werden in 700 ml Wasser gelöst und mit 1n-Natronlauge auf pH 7,6 eingestellt. Nun wird die Lösung auf 1 Liter mit Wasser aufgefüllt.

5 g 4,6-O-Benzyliden-D-galactose bzw. 4,6-O-Benzyliden-D-glucose werden in 300 ml Phosphatpuffer von pH 7,6 gelöst. Nun werden portionsweise 9,25 g (43,2 mMol) Natriumperiodat zugegeben. Durch stetiges Zutropfen von 2n-Natronlauge wird der pH zwischen 7,5 und 7,7 gehalten. (kräftig rühren). Nach der Zugabe des Natriumperiodates wird die schäumende Lösung noch 2 Stunden lang gerührt. Die klare Lösung wird nun am Wasserstrahlvakuum bis zur Trockne eingeengt. Der Rückstand wird mit Tetrahydrofuran (ca. 3 x 150 ml) extrahiert, eingeengt und am Hochvakuum getrocknet.
Ausbeute: 3,3 g (85 %) von Verbindung (2)
3,4 g (87 %) von Verbindung (14)
DC : (Toluol/Ethanol 3:1) $R_f$: 0,66
Smp. : 158 bis 161 °C für die Verbindung ( 1 )
140 bis 145 °C für die Verbindung (14)

1,3-O-Benzyliden-(2R,3S)-1,2,3-trihydroxy-4trans-octadecen -Verbindung (15)

70 g Tetradecylphosphoniumbromid wird unter Stickstoffatmosphäre in 1 Liter wasserfreiem Toluol gegeben. Es wird auf -30 °C gekühlt und nun frisch bereitetes Phenyllithium in Diethylether (aus 5,4 g Lithium und 62 g Brombenzol) unter kräftigem Rühren schnell zugetropft. Die Farbe der Lösung wird orange. Nun werden 16 g (76,8 mMol) von Verbindung (14) in Tetrahydrofuran langsam zugetropft. Nach dem Zutropfen wird 5 Minuten bei -30 °C und dann 20 Minuten bei Zimmertemperatur gerührt. Die braune Lösung wird zuerst mit Methanol verdünnt und dann wird Wasser zugegeben. Die organische Phase wird abgetrennt, am Wasserstrahlvakuum eingeengt und der Rückstand zur Reinigung auf eine Kieselgelsäule (Petrolether/Essigester 4:1) gegeben.
Ausbeute: 18 g (60 % der Theorie)
DC : (Petrolether/Essigester 4:1) $R_f$ = 0,18
Smp. : 38 bis 39 °C

| Analyse für $C_{25}H_{40}O_3$ (Mol.gew. 388,6): | | | | |
|---|---|---|---|---|
| berechnet:<br>gefunden : | C | 77,27<br>77,0<br>77,02 | H | 10,38<br>10,47<br>10,17 |

$^1$H-NMR-Spektrum ( in $CDCl_3$):
7,51 - 7,28 ( m, 5H, aromat. ); 6,0 - 5,88 ( m, 1H - C<u>H</u> = CH - OH ); 5,36 ( s, 1H, Ph - C<u>H</u> - ); 5,62 - 5,48 ( dd, 1H, CH = C<u>H</u> OH, J $_{trans}$ = 15,5 Hz, $J_{vic}$ = 6,8 ); 4,4 - 4,3 ( dd, 1H, CH = CH - C<u>H</u>O ); 3,97 - 3,93 ( m, 1H, - C<u>H</u> - OH ); 3,69 - 3,6 ( m, 2H, - C<u>H</u>$_2$ - O ); 2,14 - 2,06 ( m, 2H, CH = CH - C<u>H</u>$_2$ ); 1,75 -1,68 ( d, 1H, - OH, $J_{vic}$ = 3 Hz ); 1,5 - 1,23 ( m, 22H, aliphat. ); 0,93 - 0,85 ( t, 3H, - $CH_3$ )

2-Azido-1,3-O-benzyliden-(2S,3S)-1,3-dihydroxy-4trans-octadecen - Verbindung (16)

200 ml wasserfreies Methylenchlorid und 3,3 ml wasserfreies Pyridin werden unter Stickstoffatmosphäre auf -15 °C gekühlt. Zu dieser Lösung gibt man 12 g (31 mMol) von Verbindung (15). Nun werden 5,56 ml (35 mMol) Trifluormethansulfonsäurechloridzugetropft. Nach 5 Minuten starkem Rühren (die Lösung wird dunkelrot) wird 200 ml aminfreies Dimethylformamid und 8 g Natriumazid (ca. 10 molarer Ueberschuss) zugegeben. Nach 2 Stunden unter starkem Rühren wird die Reaktionsmischung auf ca. 200 ml Wasser gegossen und mit Petrolether (3 x 100 ml) extrahiert, über Magnesiumsulfat getrocknet und am Wasserstrahlvakuum eingeengt. Der Rückstand wird zur Reinigung über eine Kieselgelsäule (Petrolether/Essigester 9:1) gegeben.
DC : (Petrolether/Essigester 9:1) $R_f$ = 0,58
$^1$H - NMR-Spektrum (in $CDCl_3$):
7,54 - 7,38 ( m, 5H, aromat. ); 4,86 ( s, 1H, Ph - C<u>H</u> - ); 5,96 - 5,82 ( m, 1H, C<u>H</u> = CH- OH ); 5,57 - 5,44 ( dd, 1H, CH = C<u>H</u> - OH, $J_{trans}$ = 15,8 Hz, $J_{vic}$ = 7 Hz ); 4,75 - 4,65 ( dd, 1H, C<u>H</u> = CH - C<u>H</u>O, $J_1$ = $J_2$ = 6 Hz ), 4,39 - 4,28 ( m, 1H, C<u>H</u> - $N_3$ ); 3,48 - 3,38 ( dd, 1H, O - C<u>H</u>$_2$ - C ); 3,35 - 3,25 ( dd, 1H, O - C<u>H</u>$_2$ - C ); 2,18 - 2,05 ( m, 2H, CH = CH -C<u>H</u>$_2$ ); 1,45 - 1,15 ( m, 22H, aliphat. ); 0,93 - 0,85 ( t, 3H, - $CH_3$ )
Aufgrund ihrer Instabilität wurde Verbindung (16) unmittelbar zu Folgereaktionen eingesetzt.

(2S,3S)-2-Azido-1,3-dihydroxy- 4trans-octadecen -Verbindung (17)

Durchführung der Synthese wie in EP 212 400, Seite 22 für das (2S,3R)-2-Azidol-1-hydroxy-4trans-octadecen beschrieben, ausgehend von Verbindung (16).
Ausbeute: 50 mg (68 % der Theorie)
DC : (Methylenchlorid/Methanol 95:5) $R_f$ = 0,36

| Analyse für $C_{18}H_{35}O_2N_3$ (Mol.gew. 325,5): | | | | |
|---|---|---|---|---|
| berechnet:<br>gefunden : | C | 66,42<br>66,90 | H | 10,84<br>10,99 |

$^1$H - NMR-Spektrum (in $CDCl_3$):
5,86 - 5,75 ( m, 1H, - CH = CH - CHOH); 5,53 - 5,44 ( dd, 1H, - CH = CH - CHOH, $J_{trans}$ =14,5 Hz, $J_{vic}$ = 6,5 Hz ); 4,18 - 4,11 ( dd, 1H; - CH = CH - CHOH ); 3,8 - 3,73 ( m, 1H, - CH - $N_3$ ); 3,42 - 3,36 ( m, 2H, - $CH_2$ - OH ); 2,29 -2,27 ( d, 1H, - OH ); 2,17 - 2,05 ( m, 2H, CH = CH - $CH_2$ ); 1,91 - 1,89 ( d, 1H, - OH ); 1,37 - 1,2 ( m, 22H, aliphat. ); 0,98 - 0,85 ( t, 3H, - $CH_3$)

(2S,3S)-2-Amino-1,3-dihydroxy-4trans-octadecen -Verbindung (18)

50 mg (0,16 mMol) von Verbindung (17) werden in 10 ml Pyridin mit 1 ml Wasser gelöst und unter Rühren Schwefelwasserstoff eingeleitet ($H_2S$ aus NaHS). Nach 2 Stunden wird das Lösungsmittelgemisch abgezogen und der Rückstand aus wenig Acetonitril zweimal umkristallisiert.
Ausbeute: 38 mg (95 % der Theorie)
DC : (Chloroform/Methanol/2N $NH_3$ 40:10:1)
$R_f$ = 0,44
Detektion mit Ninhydrin
$^1$H - NMR-Spektrum (in $CDCl_3$):
5,88 - 5,72 ( m, 1H, - CH = CH - CHOH ); 5,55 - 5,42 ( dd, 1H, - CH = CH - CHOH, $J_{trans}$ = 15 Hz, $J_{vic}$ = 7 Hz ); 4,2 -4,2 ( dd, 1H, CH = CH - CHOH, $J_1$ = $J_2$ = 5,5 Hz ); 3,8 - 3,7 ( m, 1H - CH - $NH_2$ ); 3,45 - 3,35 ( m, 2H, - $CH_2$ - OH); 1,85 - 1,6 (m, 6H, CH = CH - $CH_2$ - $NH_2$, 2 mal - OH ); 1,42 -1,11 ( m, 22H, aliphat. ); 0,95 - 0,85 ( t, 3H, - $CH_3$ )

Umsetzung mit einem Phosphonat

a) Tetradecan-1-phosphonsäurediethylester

100 g (590 mMol) Triethylphosphit und 164 g (590 mMol) 1-Bromtetradecan werden an einer mit absteigendem Kühler verbundenen Kolonne erhitzt. Bei einer Badtemperatur von 170 °C destilliert Bromethan ab; anschliessend wird im Hochvakuum destilliert und dadurch das $^1$H-NMR-reine Produkt vom Siedepunkt 145 °C/0,03 mm Hg erhalten.

b) (2S,3R,4E)-1,3-O-Benzyllden-1,2,3-trihydroxy-4-octadecen

16,6 g (50 mMol) Tetradecan-1-phosphonsäure-diethylester werden in 10 ml wasserfreiem Tetrahydrofuran gelöst und mit Schutzgasatmosphäre auf -40 °C gekühlt. Zum entstandenen heterogenen Gemisch werden 1,6 g(25 mMol, in Tetrahydrofuran) n-Butyllithium langsam zugetropft und gleichzeitig die Temperatur auf -50 °C erniedrigt. Anschliessend werden 10 g (46,6 mMol) 2,4-O-Benzyliden-D-threose, gelöst in 10 ml Tetrahydrofuran, langsam zugetropft. Nach 1 Stunde (DC-Kontrolle) wird auf Zimmertemperatur erwärmt und die Reaktionslösung zu 20 ml Wasser gegeben. Die Phasen werden getrennt, die organische Phase über Magnesiumsulfat getrocknet und im Vakuum eingeengt. Das erhaltene Oel löst man in 5 ml Essigester und versetzt mit mehrmals mit Methanol säurefrei gewaschenem saurem Ionenaustauscher (Amberlite IR 120). Nach 5 Stunden Rühren bei Zimmertemperatur ist die Eliminierungsreaktion beendet. Man filtriert den Ionenaustauscher ab, engt ein und chromatographiert (Petrolether/Essigester 9:1).
DC : (Petrolether/Essigester 9:1) $R_f$ = 0,21
Das erhaltene Produkt ist mit der nach R.R. Schmidt et al., Tetrahedron Letters 27 (1986), 481, erhaltenen Verbindung $^1$H-NMR-spektroskopisch identisch.

## Patentansprüche

## Patentansprüche für folgende Vertragsstaaten : BE, CH, DE, FR, GB, IT, LI, LU, NL, SE

1. Verwendung einer Lactosylverbindung der allgemeinen Formel I nach Formelblatt, in welcher Formel $R^1$ einen aliphatischen Rest mit 9 bis 19 Kohlenstoffatomen in gerader Kette, welche eine oder mehrere Doppelbindungen oder/und seitliche Methylgruppen tragen kann, $R^2$ den Acylrest einer gesättigten oder einer einfach oder mehrfach ungesättigten Fettsäure mit 14 bis 24 Kohlenstoffatomen und X eine Gruppe der Formel $-CH_2-CH_2$ - oder -CH=CH- bedeutet, zusammen mit üblichen Excipientien und Hilfsstoffen zur Herstellung eines zytoprotektiven Arzneimittels, insbesondere gegen zell- und gewebeschädigende entzündliche Einwirkungen.

2. Verwendung nach Anspruch 1, dadurch gekennzeichnet, dass der aliphatische Rest $R^1$ der Lactosylverbindung 13 bis 15 Kohlenstoffatome in gerader Kette umfasst.

3. Verwendung nach Anspruch 1, dadurch gekennzeichnet, dass der Acylrest $R^2$ der Lactosylverbindung eine gerade Anzahl Kohlenstoffatome umfasst.

4. Verwendung nach Anspruch 1, dadurch gekennzeichnet, dass der Acylrest $R^2$ der Lactosylverbindung 16, 18 oder 20 Kohlenstoffatome in gerader Kette umfasst.

5. Lactosylverbindungen der allgemeinen Formel IA nach Formelblatt, in welcher $R^1$ und X dieselbe Bedeutung wie im Anspruch 1 haben und $R^{2'}$ den Acylrest einer einfach oder mehrfach ungesättigten Fettsäure mit 14 bis 24 Kohlenstoffatomen bedeutet.

6. Lactosylverbindungen nach Anspruch 5, in welchen der aliphatische Rest $R^1$ 13 bis 15 Kohlenstoffatome in gerader Kette umfasst.

7. Lactosylverbindungen nach Anspruch 5, in welchen der Acylrest $R^{2'}$ eine gerade Anzahl Kohlenstoffatome umfasst.

8. Lactosylverbindungen nach Anspruch 7, in welchen der Acylrest $R^{2'}$ 16, 18 oder 20 Kohlenstoffatome in gerader Kette umfasst.

9. Verfahren zur Herstellung der Lactosylverbindungen nach Anspruch 5, dadurch gekennzeichnet, dass man eine Azidoverbindung der Formel II, in welcher R Wasserstoff oder eine Schutzgruppe bedeutet und $R^1$ obige Bedeutung hat, mit dem O-Trifluor- oder O-Trichlor-acetimidat oder dem 1-Halogenderivat oder 1-Thioderivat einer Lactose, deren Hydroxylgruppen in den 2,3,6,2',3',4',6'-Stellungen durch Acylgruppen Ac geschützt sind, umsetzt, von der erhaltenen Verbindung der Formel III die Acylgruppen Ac und, wenn vorhanden, die Schutzgruppe R abspaltet, in der erhaltenen Verbindung der Formel IV die Azidogruppe in eine primäre Aminogruppe überführt und die erhaltene Verbindung der Formel V einer N-Acylierung mit einer Fettsäure $R^{2'}$-OH oder einem reaktionsfähigen Derivat derselben unterwirft.

10. Verfahren nach Anspruch 9, dadurch gekennzeichnet,dass als Hydroxylschutzgruppe R die Acylgruppe einer aliphatischen oder aromatischen Carbonsäure oder eine tert.-Butoxycarbonylgruppe, vorzugsweise die Acylgruppe der Benzoesäure oder einer substituierten Benzoesäure oder der Pivalinsäure, verwendet wird.

11. Verfahren nach Anspruch 9, dadurch gekennzeichnet, dass die Umsetzung der Verbindung der Formel II mit besagtem O-Trifluor- oder O-Trichlor-acetimidat in Gegenwart eines Lewis-Säure-Katalysators und in einem wasserfreien Kohlenwasserstoff oder halogenierten Kohlenwasserstoff, jene mit besagtem 1-Halogenderivat in Gegenwart eines säurebindenden Mittels oder eines Schwermetallsalzes und jene mit besagtem 1-Thioderivat in Gegenwart einer Säure durchgeführt wird.

12. Verfahren nach Anspruch 9, dadurch gekennzeichnet, dass die Acylgruppen Ac und die Schutzgruppe R von der Verbindung der Formel III durch basische Katalyse abgespalten werden.

13. Verfahren nach Anspruch 9, dadurch gekennzeichnet, dass die Ueberführung der Azidogruppe der Verbindung der Formel IV in eine primäre Aminogruppe durch Behandlung mit Schwefelwasserstoff in

einem Gemisch (1:1) von Wasser und Pyridin oder durch Hydrierung mit Natriumborhydrid oder einem anderen Reduktionsmittel durchgeführt wird.

**14.** Verfahren nach Anspruch 9, dadurch gekennzeichnet, dass die N-Acylierung der Verbindung der Formel V mittels der Fettsäure der Formel $R^{2'}$-OH in Gegenwart eines wasserabspaltenden Mittels oder mittels eines aktivierten Esters der Fettsäure oder mittels eines Halogenids derselben in Gegenwart einer anorganischen Base oder einer tertiären organischen Base durchgeführt wird.

**Patentansprüche für folgende Vertragsstaaten : AT, ES, GR**

**1.** Verwendung einer Lactosylverbindung der allgemeinen Formel I nach Formelblatt, in welcher Formel $R^1$ einen aliphatischen Rest mit 9 bis 19 Kohlenstoffatomen in gerader Kette, welche eine oder mehrere Doppelbindungen oder/und seitliche Methylgruppen tragen kann, $R^2$ den Acylrest einer gesättigten oder einer einfach oder mehrfach ungesättigten Fettsäure mit 14 bis 24 Kohlenstoffatomen und X eine Gruppe der Formel $-CH_2-CH_2-$ oder $-CH=CH-$ bedeutet, zusammen mit üblichen Excipientien und Hilfsstoffen zur Herstellung eines zytoprotektiven Arzneimittels, insbesondere gegen zell- und gewebeschädigende entzündliche Einwirkungen.

**2.** Verwendung nach Anspruch 1, dadurch gekennzeichnet, dass der aliphatische Rest $R^1$ der Lactosylverbindung 13 bis 15 Kohlenstoffatome in gerader Kette umfasst.

**3.** Verwendung nach Anspruch 1, dadurch gekennzeichnet, dass der Acylrest $R^2$ der Lactosylverbindung eine gerade Anzahl Kohlenstoffatome umfasst.

**4.** Verwendung nach Anspruch 1, dadurch gekennzeichnet, dass der Acylrest $R^2$ der Lactosylverbindung 16, 18 oder 20 Kohlenstoffatome in gerader Kette umfasst.

**5.** Verfahren zur Herstellung der Lactosylverbindungen der allgemeinen Formel IA nach Formelblatt, in welcher $R^1$ und X dieselbe Bedeutung wie in Anspruch 1 haben und $R^{2'}$ den Acylrest einer einfach oder mehrfach ungesättigten Fettsäure mit 14 mit 24 Kohlenstoffatomen bedeutet, dadurch gekennzeichnet, dass man eine Azidoverbindung der Formel II, in welcher R Wasserstoff oder eine Schutzgruppe bedeutet und $R^1$ obige Bedeutung hat, mit dem O-Trifluor- oder O-Trichlor-acetimidat oder dem 1-Halogenderivat oder 1-Thioderivat einer Lactose, deren Hydroxylgruppen in den 2,3,6,2',3',4',6'-Stellungen durch Acylgruppen Ac geschützt sind, umsetzt, von der erhaltenen Verbindung der Formel III die Acylgruppen Ac und, wenn vorhanden, die Schutzgruppe R abspaltet, in der erhaltenen Verbindung der Formel IV die Azidogruppe in eine primäre Aminogruppe überführt und die erhaltene Verbindung der Formel V einer N-Acylierung mit einer Fettsäure $R^{2'}$-OH oder einem reaktionsfähigen Derivat derselben unterwirft.

**6.** Verfahren nach Anspruch 5, dadurch gekennzeichnet, dass als Hydroxylschutzgruppe R die Acylgruppe einer aliphatischen oder aromatischen Carbonsäure oder eine tert.-Butoxycarbonylgruppe, vorzugsweise die Acylgruppe der Benzoesäure oder einer substituierten Benzoesäure oder der Pivalinsäure, verwendet wird.

**7.** Verfahren nach Anspruch 5, dadurch gekennzeichnet, dass die Umsetzung der Verbindung der Formel II mit besagtem O-Trifluor- oder O-Trichlor-acetimidat in Gegenwart eines Lewis-Säure-Katalysators und in einem wasserfreien Kohlenwasserstoff oder halogenierten Kohlenwasserstoff, jene mit besagtem 1-Halogenderivat in Gegenwart eines säurebindenden Mittels oder eines Schwermetallsalzes und jene mit besagtem 1-Thioderivat in Gegenwart einer Säure durchgeführt wird.

**8.** Verfahren nach Anspruch 5, dadurch gekennzeichnet, dass die Acylgruppen Ac und die Schutzgruppe R von der Verbindung der Formel III durch basische Katalyse abgespalten werden.

**9.** Verfahren nach Anspruch 5, dadurch gekennzeichnet, dass die Ueberführung der Azidogruppe der Verbindung der Formel IV in eine primäre Aminogruppe durch Behandlung min Schwefelwasserstoff in einem Gemisch (1:1) von Wasser und Pyridin oder durch Hydrierung mit Natriumborhydrid oder einem anderen Reduktionsmittel durchgeführt wird.

10. Verfahren nach Anspruch 5, dadurch gekennzeichnet, dass die N-Acylierung der Verbindung der Formel V mittels der Fettsäure der Formel $R^{2'}$-OH in Gegenwart eines wasserabspaltenden Mittels oder mittels eines aktivierten Esters der Fettsäure oder mittels eines Halogenids derselben in Gegenwart einer anorganischen Base oder einer tertiären organischen Base durchgeführt wird.

**Claims**
**Claims for the following Contracting States : BE, CH, DE, FR, GB, IT, LI, LU, NL, SE**

1. The use of a lactosyl compound of the general formula I shown in the formula sheet, in which formula $R^1$ denotes an aliphatic radical which has 9 to 19 carbon atoms in the straight chain and which can have one or more double bonds and/or branching methyl groups, $R^2$ denotes the acyl radical of a saturated or singly or multiply unsaturated fatty acid having 14 to 24 carbon atoms, and X denotes a group of the formula $-CH_2-CH_2-$or$-CH=CH-$, together with customary excipients and auxiliaries for the preparation of a cytoprotective agent, especially to counter cell- and tissue-damaging inflammatory actions.

2. A use as claimed in claim 1, in which the aliphatic radical $R^1$ in the lactosyl compound therein has 13 to 15 carbon atoms in the straight chain.

3. A use as claimed in claim 1, in which the acyl radical $R^2$ in the lactosyl compound therein has an even number of carbon atoms.

4. A use as claimed in claim 1, in which the acyl radical $R^2$ in the lactosyl compound therein has 16, 18 or 20 carbon atoms in the straight chain.

5. A lactosyl compound of the general formula IA shown in the formula sheet, in which $R^1$ and X have the same meaning as in claim 1, and $R^{2'}$ denotes the acyl radical of a singly or multiply unsaturated fatty acid having 14 to 24 carbon atoms.

6. A lactosyl compound as claimed in claim 5, in which the aliphatic radical $R^1$ has 13 to 15 carbon atoms in the straight chain.

7. A lactosyl compound as claimed in claim 5, in which the acyl radical $R^{2'}$ has an even number of carbon atoms.

8. A lactosyl compound as claimed in claim 7, in which the acyl radical $R^{2'}$ has 16, 18 or 20 carbon atoms in the straight chain.

9. A process for the preparation of a lactosyl compound as claimed in claim 5, which comprises reacting an azido compound of the formula II in which R denotes hydrogen or a protective group, and $R^1$ has the above meaning, with the O-trifluoro- or O-trichloro-acetimidate or the 1-halogeno derivative or 1-thio derivative of a lactose whose hydroxyl groups in the 2, 3, 6, 2', 3', 4' and 6' positions are protected by acyl groups Ac, eliminating from the resulting compound of the formula III the acyl groups Ac and, where present, the protective group R, converting the azido group in the resulting compound of the formula IV into a primary amino group, and subjecting the resulting compound of the formula V to an N-acylation with fatty acid $R^{2'}$-OH or a reactive derivative thereof.

10. The process as claimed in claim 9, wherein the hydroxyl protective group R used is the acyl group of an aliphatic or aromatic carboxylic acid or a tert.-butoxycarbonyl group, preferably the acyl group of benzoic acid or of a substituted benzoic acid or of pivalic acid.

11. The process as claimed in claim 9, wherein the reaction of the compound of the formula II with the said O-trifluoro- or O-trichloro-acetimidate is carried out in the presence of a Lewis acid catalyst and in an anhydrous hydrocarbon or halogenated hydrocarbon, and that with the said 1- halogeno derivative is carried out in the presence of an acid-binding agent or of a heavy metal salt, and that with the said 1-thio derivative is carried out in the presence of an acid.

12. The process as claimed in claim 9, wherein the acyl groups Ac and the protective group R are eliminated from the compound of the formula III by basic catalysis.

13. The process as claimed in claim 9, wherein the conversion of the azido group of the compound of the formula IV into a primary amino group is carried out by treatment with hydrogen sulfide in a mixture (1:1) of water and pyridine or by hydrogenation with sodium borohydride or another reducing agent.

14. The process as claimed in claim 9, wherein the N-acylation of the compound of the formula V with the fatty acid of the formula $R^{2'}$-OH is carried out in the presence of a water-removing agent or using an activated ester of the fatty acid or using a halide thereof, in the presence of an inorgaic base or a tertiary organic base.

**Claims for the following Contracting States : AT, ES, GR**

1. The use of a lactosyl compound of the general formula I shown in the formula sheet, in which formula $R^1$ denotes an aliphatic radical which has 9 to 19 carbon atoms in the straight chain and which can have one or more double bonds and/or branching methyl groups, $R^2$ denotes the acyl radical of a saturated or singly or multiply unsaturated fatty acid having 14 to 24 carbon atoms, and X denotes a group of the formula $-CH_2-CH_2-$or$-CH=CH-$, together with customary excipients and auxiliaries for the preparation of a cytoprotective agent, especially to counter cell- and tissue-damaging inflammatory actions.

2. A use as claimed in claim 1, in which the aliphatic radical $R^1$ in the lactosyl compound therein has 13 to 15 carbon atoms in the straight chain.

3. A use as claimed in claim 1, in which the acyl radical $R^2$ in the lactosyl compound therein has an even number of carbon atoms.

4. A use as claimed in claim 1, in which the acyl radical $R^2$ in the lactosyl compound therein has 16, 18 or 20 carbon atoms in the straight chain.

5. A process for the preparation of a lactosyl compound of the general formula IA shown in the formula sheet, in which $R^1$ and X have the same meaning as in claim 1, and $R^{2'}$ denotes the acyl radical of a singly or multiply unsaturated fatty acid having 14 to 24 carbon atoms, which comprises reacting an azido compound of the formula II in which R denotes hydrogen or a protective group, and $R^1$ has the above meaning, with the O-trifluoro- or O-trichloro-acetimidate or the 1-halogeno derivative or 1-thio derivative of a lactose whose hydroxyl groups in the 2, 3, 6, 2', 3', 4' and 6' positions are protected by acyl groups Ac, eliminating from the resulting compound of the formula III the acyl groups Ac and, where present, the protective group R, converting the azido group in the resulting compound of the formula IV into a primary amino group, and subjecting the resulting compound of the formula V to an N-acylation with a fatty acid $R^{2'}$-OH or a reactive derivative thereof.

6. The process as claimed in claim 5, wherein the hydroxyl protective group R used is the acyl group of an aliphatic or aromatic carboxylic acid or a tert.-butoxycarbonyl group, preferably the acyl group of benzoic acid or of a substituted benzoic acid or of pivalic acid.

7. The process as claimed in claim 5, wherein the reaction of the compound of the formula II with the said O-trifluoro- or O-trichloro-acetimidate is carried out in the presence of a Lewis acid catalyst and in an anhydrous hydrocarbon or halogenated hydrocarbon, and that with the said 1-halogeno derivative is carried out in the presence of an acid-binding agent or of a heavy metal salt, and that with the said 1-thio derivative is carried out in the presence of an acid.

8. The process as claimed in claim 5, wherein the acyl groups Ac and the protective group R are eliminated from the compound of the formula III by basic catalysis.

9. The process as claimed in claim 5, wherein the conversion of the azido group of the compound of the formula IV into a primary amino group is carried out by treatment with hydrogen sulfide in a mixture (1:1) of water and pyridine or by hydrogenation with sodium borohydride or another reducing agent.

10. The process as claimed in claim 5, wherein the N-acylation of the compound of the formula V with the fatty acid of the formula $R^{2'}$-OH is carried out in the presence of a water-removing agent or using an activated ester of the fatty acid or using a halide thereof, in the presence of an inorganic base or a tertiary organic base.

**Revendications**

**Revendications pour les Etats contractants suivants : BE, CH, DE, FR, GB, IT, LI, LU, NL, SE**

1. Utilisation d'un composé lactosyle de formule générale I selon la planche de formules, formule dans laquelle $R^1$ représente un radical aliphatique comportant de 9 à 19 atomes de carbone en chaîne droite, chaîne qui peut porter une ou plusieurs doubles liaisons ou/et groupes méthyles latéraux, $R^2$ représente le radical acyle d'un acide gras saturé ou une ou plusieurs fois insaturé comportant de 14 à 24 atomes de carbone et X représente un groupe de formule $-CH_2-CH_2-$ou $-CH=CH-$, ensemble avec des excipients et adjuvants usuels pour la préparation d'un médicament cytoprotecteur, notamment contre des actions inflammatoires dommageables pour les cellules et les tissus.

2. Utilisation selon la revendication 1, caractérisée en ce que le radical aliphatique $R^1$ du composé lactosyle comprend de 13 à 15 atomes de carbone en chaîne droite.

3. Utilisation selon la revendication 1, caractérisée en ce que le radical acyle $R^2$ du composé lactosyle comprend un nombre pair d'atomes de carbone.

4. Utilisation selon la revendication 1, caractérisée en ce que le radical acyle $R^2$ du composé lactosyle comporte 16, 18 ou 20 atomes de carbone en chaîne droite.

5. Composés lactosyles de formule générale IA selon la planche de figures, dans laquelle $R^1$ et X ont la même signification qu'à la revendication 1 et $R^{2'}$ représente le radical acyle d un acide gras une ou plusieurs fois insaturé comportant de 14 à 24 atomes de carbone.

6. Composés lactosyles selon la revendication 5, dans lesquels le radical aliphatique $R^1$ comporte de 13 à 15 atomes de carbone en chaîne droite.

7. Composés lactosyles selon la revendication 5, dans lesquels le radical acyle $R^{2'}$ comprend un nombre pair d'atomes de carbone.

8. Composés lactosyles selon la revendication 7, dans lesquels le radical acyle $R^{2'}$ comporte 16, 18 ou 20 atomes de carbone en chaîne droite.

9. Procédé de préparation des composés lactosyles selon la revendication 5, caractérisé en ce que l'on fait réagir un composé azido de formule II, dans laquelle R représente l'hydrogène ou un groupe protecteur et $R^1$ a la signification indiquée ci-dessus, avec le O-trifluoro- ou le O-trichloro-acétimidate ou le dérivé 1-halogéno ou le dérivé 1-thio d'un lactose dont les groupes hydroxyles en les positions 2,3,6,2',3',4',6' sont protégés par des groupes acyles Ac, on scinde du composé obtenu, de formule III, les groupes acyles Ac et, lorsqu'il est présent, le groupe protecteur R, on transforme le groupe azido dans le composé obtenu de formule IV en un groupe amino primaire et on soumet le composé obtenu de formule V à une N-acylation par un acide gras $R^{2'}$-OH ou par un dérivé réactif de celui-ci.

10. Procédé selon la revendication 9, caractérisé en ce qu'on utilise comme groupe protecteur de l'hydroxyle R le groupe acyle d'un acide carboxylique aliphatique ou aromatique ou un groupe tert.butoxycarbonyle, de préférence, le groupe acyle de l'acide benzoïque ou d'un acide benzoïque substitué ou de l'acide pivalique.

11. Procédé selon la revendication 9, caractérisé en ce que la réaction du composé de formule II avec ledit O-trifluoro- ou O-trichloro-acétimidate est effectuée en présence d'un acide de Lewis comme catalyseur et dans un hydrocarbure ou un hydrocarbure halogéné exempts d'eau, la réaction avec ledit dérivé 1-halogéno est effectuée en présence d'un agent accepteur d'acide ou d'un sel de métal lourd et la réaction avec ledit dérivé 1-thio est effectuée en présence d'un acide.

**12.** Procédé selon la revendication 9, caractérisé en ce que les groupes acyles Ac et le groupe protecteur R sont scindés du composé de formule III par catalyse basique.

**13.** Procédé selon la revendication 9, caractérisé en ce que la transformation du groupe azido du composé de formule IV en un groupe amino primaire est effectuée par traitement au moyen d'hydrogène sulfuré dans un mélange (1:1) d'eau et de pyridine ou par hydrogénation au moyen d'hydrure de sodium et de bore ou d'un autre agent réducteur.

**14.** Procédé selon la revendication 9, caractérisé en ce que la N-acylation du composé de formule V au moyen de l'acide gras de formule $R^{2'}$-OH est effectuée en présence d'un agent d'élimination d'eau ou au moyen d'un ester activé de l'acide gras ou au moyen d'un halogénure du même en présence d'une base minérale ou d'une base organique tertiaire.

**Revendications pour les Etats contractants suivants : AT, ES, GR**

**1.** Utilisation d un composé lactosyle de formule générale I selon la planche de formules, formule dans laquelle $R^1$ représente un radical aliphatique comportant de 9 à 19 atomes de carbone en chaîne droite, chaîne qui peut porter une ou plusieurs doubles liaisons ou/et groupes méthyles latéraux, $R^2$ représente le radical acyle d'un acide gras saturé ou une ou plusieurs fois insaturé comportant de 14 à 24 atomes de carbone et X représente un groupe de formule $-CH_2-CH_2-$ou $-CH=CH-$, ensemble avec des excipients et adjuvants usuels pour la préparation d'un médicament cytoprotecteur, notamment contre des actions inflammatoires dommageables pour les cellules et les tissus.

**2.** Utilisation selon la revendication 1, caractérisée en ce que le radical aliphatique $R^1$ du composé lactosyle comprend de 13 à 15 atomes de carbone en chaîne droite.

**3.** Utilisation selon la revendication 1, caractérisée en ce que le radical acyle $R^2$ du composé lactosyle comprend un nombre pair d'atomes de carbone.

**4.** Utilisation selon la revendication 1, caractérisée en ce que le radical acyle $R^2$ du composé lactosyle comporte 16, 18 ou 20 atomes de carbone en chaîne droite.

**5.** Procédé de préparation des composés lactosyles de formule générale IA selon la planche de formules, dans laquelle $R^1$ et X ont la même signification qu'à la revendication 1 et $R^{2'}$ représente le radical acyle d'un acide gras une ou plusieurs fois insaturé comportant de 14 à 24 atomes de carbone, caractérisé en ce que l'on fait réagir un composé azido de formule II, dans laquelle R représente l'hydrogène ou un groupe protecteur et $R^1$ a la signification indiquée ci-dessus, avec le O-trifluoro- ou le O-trichloro-acétimidate ou le dérivé 1-halogéno ou le dérivé 1-thio d'un lactose dont les groupes hydroxyles en les positions 2,3,6,2',3',4',6' sont protégés par des groupes acyles Ac, on scinde du composé obtenu, de formule III, les groupes acyles Ac et, lorsqu'il est présent, le groupe protecteur R, on transforme le groupe azido dans le composé obtenu de formule IV en un groupe amino primaire et on soumet le composé obtenu de formule V à une N-acylation par un acide gras $R^{2'}$-OH ou par un dérivé réactif de celui-ci.

**6.** Procédé selon la revendication 5, caractérisé en ce qu'on utilise comme groupe protecteur de l'hydroxyle R le groupe acyle d'un acide carboxylique aliphatique ou aromatique ou un groupe tert.butoxycarbonyle, de préférence, le groupe acyle de l'acide benzoïque ou d'un acide benzoïque substitué ou de l'acide pivalique.

**7.** Procédé selon la revendication 5, caractérisé en ce que la réaction du composé de formule II avec ledit O-trifluoro- ou O-trichloro-acétimidate est effectuée en présence d'un acide de Lewis comme catalyseur et dans un hydrocarbure ou un hydrocarbure halogéné exempts d'eau, la réaction avec ledit dérivé 1-halogéno est effectuée en présence d'un agent accepteur d'acide ou d'un sel de métal lourd et la réaction avec ledit dérivé 1-thio est effectuée en présence d'un acide.

**8.** Procédé selon la revendication 5, caractérisé en ce que les groupes acyles Ac et le groupe protecteur R sont scindés du composé de formule III par catalyse basique.

**9.** Procédé selon la revendication 5, caractérisé en ce que la transformation du groupe azido du composé de formule IV en un groupe amino primaire est effectuée par traitement au moyen d'hydrogène sulfuré dans un mélange (1:1) d'eau et de pyridine ou par hydrogénation au moyen d'hydrure de sodium et de bore ou d'un autre agent réducteur.

**10.** Procédé selon la revendication 5, caractérisé en ce que la N-acylation du composé de formule V au moyen de l'acide gras de formule $R^{2'}$-OH est effectuée en présence d'un agent d'élimination d'eau ou au moyen d'un ester activé de l'acide gras ou au moyen d'un halogénure du même en présence d'une base minérale ou d'une base organique tertiaire.

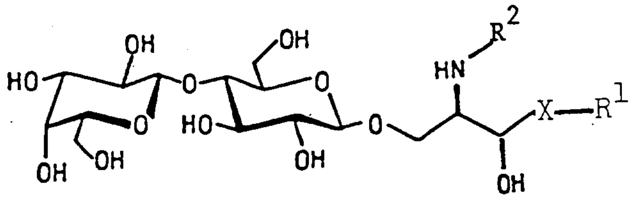

(I)

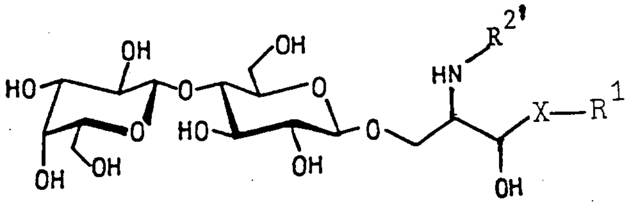

(IA)

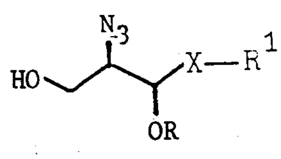

(II)

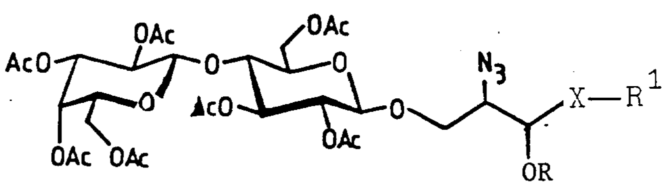

(III)

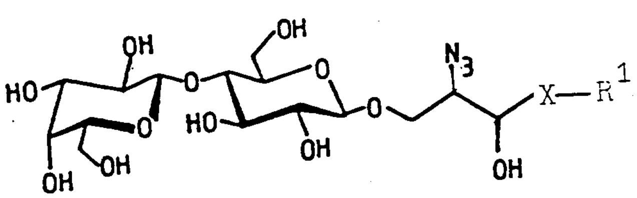

(IV)

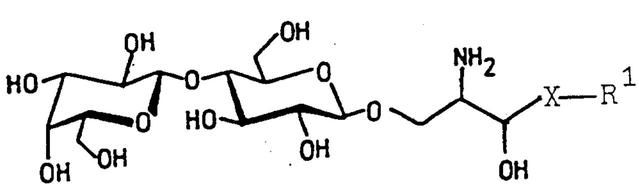

(V)